# EUROPÄISCHE PATENTSCHRIFT

(11) **EP 2 428 503 B1**
(45) Veröffentlichungstag und Bekanntmachung des Hinweises auf die Patenterteilung: **10.12.2014**
(21) Anmeldenummer: 10176289.6
(22) Anmeldetag: 10.09.2010
(51) Int. Cl.: C07C 271/22, C07C 231/14, C07C 233/18, C07C 237/06, C07C 237/08, C07C 231/02

(54) **Synthese von tripodalen Catecholderivaten mit einem flexiblen Grundgerüst zur Funktionalisierung von Oberflächen**
Synthesis of tripodal catechol derivatives with a flexible base for functionalising surfaces
Synthèse de dérivés de catéchol tripodaux flexibles pour la fonctionnalisation de surfaces

(43) Veröffentlichungstag der Anmeldung: 14.03.2012
(73) Patentinhaber: Justus-Liebig-Universität Gießen, 35390 Gießen (DE)
(72) Erfinder: Maison, Wolfgang, Prof., 35580 Wetzlar (DE); Khalil, Faiza, 35325 Mücke (DE); Franzmann, Elisa, 35435 Wettenberg-Wißmar (DE)
(74) Vertreter: Stumpf, Peter

(56) Entgegenhaltungen:
- WO-A1-2010/037044
- WO-A2-2005/118831
- US-A1- 2008 247 984
- CHRISTOPHE HENRY ET.AL.: "New 8-hydroxyquinoline and catecholate iron chelators: Influence of their partition coefficient on their biological activity", BIOCHEMICAL PHARMACOLOGY, Bd. 62, 15. Dezember 2001 (2001-12-15), Seiten 1355-1362, XP002622354,
- SUBAN K. SAHOO ET.AL.: "Complexation of a tripodal amine-catechol ligand tris((2,3.dihydroxybenzylamino)ethyl)amine towards Al(III), Ga(III), and In(III)", MONATSH. CHEM., Bd. 140, 14. Oktober 2008 (2008-10-14), Seiten 139-145, XP002622355,
- RICHARD J. STONARD ET.AL.: "Linear peptide alkaloids from the sponge Cliona celata (Grant). Celeamides Cand D", CANADIAN JOURNAL OF CHEMISTRY, Bd. 58, Nr. 20, 15. Oktober 1980 (1980-10-15), Seiten 2121-2126, XP002622356,

## Beschreibung

Die vorliegende Erfindung beschreibt tripodale Catecholderivate mit einem flexiblen Grundgerüst zur Funktionalisierung von Oberflächen, Verfahren zu ihrer Herstellung sowie ihre Verwendung. Zentralatom der flexiblen Gerüste ist dabei ein tertiäres aliphatisches Kohlenstoffatom. Das Design der Verbindungen erfolgt biomimetisch und orientiert sich an Muscheladhäsionsproteinen und Siderophoren, die natürlicherweise hochaffine Bindungen an Oberflächen bewirken. Eine vierte verbliebene Position des flexiblen Gerüstes kann optional über sog. Click-Reaktionen beispielsweise mit Biomolekülen, Polyethylenglykol oder Wirkstoffen funktionalisiert werden.

### Beschreibung und Einleitung des allgemeinen Gebietes der Erfindung

Die vorliegende Erfindung betrifft die Gebiete organische Chemie und Materialwissenschaften.

### Stand der Technik

Der Stand der Technik (US 2008/247984) kennt zahlreiche Methoden zur Funktionalisierung von Oberflächen. Solche Funktionalisierungen werden verwendet, um die Materialeigenschaften der Oberflächen gezielt zu verändern. Derartige Funktionalisierungen sollten möglichst beständig sein und eine hohe definierte Beladung der Oberfläche erlauben.

Im Bereich der Medizintechnik kommt funktionalisierten Oberflächen eine besondere Bedeutung zu. So sollten Implantate - beispielsweise im Dentalbereich und der Orthopädie (Gelenkersatz) möglichst biokompatibel sein, d.h. unter Anderem nicht zum Biofouling neigen, keine Entzündungsreaktionen hervorrufen und nicht von pathogenen Mikroorganismen besiedelt werden. Des Weiteren müssen sie starker mechanischer Beanspruchung dauerhaft standhalten.

Medizinische Implantate enthalten häufig die Metalle Eisen und/oder Titan, Zahnimplantate zudem Apatit. Bislang wurden monomere Derivate des Catecholamins als Oberflächenbinder eingesetzt, an die dann verschiedene funktionelle Moleküle wie z.B. Antibiotika oder PEG gebunden wurden. Mit solchen Konjugaten konnte eine erhöhte Resistenz der Oberflächen gegen Biofouling nachgewiesen werden. Eine Alternative zu monomeren Catecholderivaten sind polymere Strukturen, die Muscheladhäsionsproteine imitieren. Diese kommen als biomimetische Klebstoffe zum Einsatz.

Mit Hilfe monomerer Catecholderivate lassen sich Metalloberflächen sehr einfach, aber leider auch nur wenig dauerhaft funktionalisieren. Dies ist insbesondere bei starker Materialbeanspruchung wie z.B. im Dentalbereich von Nachteil. Polymere Strukturen ermöglichen zwar eine sehr feste Verbindung, erlauben aber keine zielgerichtete und definierte Funktionalisierung der Oberfläche, wie sie z.B. für Implantate wünschenswert ist.

Methoden, mit denen Oberflächen durch Naturstoffhybride antimikrobiell beschichtet werden können, sind in J-Y Wach, S Bonazzi, K Gademann: "Antimikrobielle Oberflächen durch Naturstoffhybride", Angew Chem 2008, 120, 7232-7235 beschrieben. Die Naturstoffhybride enthalten lediglich monomere Catecholamine.

Untersuchungen zu Struktur-Wirkungsbeziehungen methylierter oder hydroxyterminierter Polyglycerol-Strukturen, die als SAMs auf Goldoberflächen aufgebracht wurden, werden in M Wyszogrodzka, R Haag: "Study of Singel Protein Adsorption onto Monoamino Oligoglycerol Derivatives: A Structure#Activity Relationship", Langmuir 2009, 25, 5703-5712 beschrieben. Die gezeigten dendritischen Polyglycerol-Strukturen enthalten keinerlei aromatische funktionelle Gruppen.

In JK Young, GR Baker, GR Newkome: "Smart Cascade Polymers. Modular Syntheses of Four-Directional Dendritic Macromolecules with Acidic, Neutral, or Basic Terminal Groups and the Effect of ph Changeson their Hydrodynamic Radii", Macromolecules 1994, 27, 3464-3471 werden Beispiele für die Synthese vierdirektionaler, flexibler dendritischer Kaskadenpolymere beschrieben. Die offenbarten dendritischen Moleküle enthalten jedoch keine Catecholamingruppen, wie sie für Muscheladhäsionsproteine charakteristisch sind.

In K Yoon, P Goyal, M Weck: "Monofunctionalization of Dendrimers with Use of Microwave-Assisted 1,3-Dipolar Cycloadditions", Org Lett 2007, 9, 2051-2054, werden ebenfalls Verfahren zur Herstellung flexibler dendritischer Verbindungen beschrieben. Die Arbeit offenbart u.a. die Einführung cyclischer funktioneller Gruppen über 1,3-dipolare Cycloadditionen. M Kleinert, T Winkler, A Terfort und TB Lindhorst beschreiben in "A modular approach for the construction and modification of glyco-SAMs utilizing 1,3-dipolar cycloaddition", Org Biomol Chem 2008, 6, 2118-2132 ebenfalls Methoden zur modularen Synthese flexibler dendritischer Verbindungen. Neben 1,3-dipolaren Cycloadditionen werden auch Clickreaktion auf SAMs beschrieben.

In SK Sahoo, BK Kanungo, M Baral: "Complexation of a tripodal amine-catechol ligand tris(2,3-dihydroxybenzylamino)ethyl)amine towards AI(III), Ga(III), and In(III), Monatsh Chem 2009, 140, 139-145 werden trivalente flexible Gerüste mit Catecholaminliganden beschrieben. Bei den trivalenten flexiblen Gerüsten handelt es sich um tertiäre aliphatische Amine, und die Catecholamine besitzen ein 2,3-Dihydroxysubstitutionsmuster. Die offenbarten Verbindungen erlauben keine Funktionalisierung mit Effektoren und sind daher für die Funktionalisierung von Oberflächen nur bedingt nutzbar. Auch bei der Nutzung der Verbindungen als Metallchelatoren lässt sich keine weitere Funktionalität, beispielsweise für die Zell-oder Pathogenerkennung, einführen.

Die vorliegende Erfindung stellt dagegen trivalente flexible Gerüste mit Liganden umfassend Catecholeinheiten bereit. Das Design dieser Verbindungen erfolgt hierbei biomimetisch und orientiert sich an Muscheladhäsionsproteinen und Siderophoren, die natürlicherweise hochaffine Bindungen an Oberflächen bewirken. Die erfindungsgemäßen Verbindungen bestehen aus tripodalen flexiblen Gerüsten mit einem tertiären aliphatischen Kohlenstoffatom, an das drei Catecholeinheiten gebunden sind. Der verbleibende vierte Substituent des tertiären Kohlenstoffatoms kann leicht über sog. Click-Reaktionen weiter funktionalisiert werden, beispielsweise mit Biomolekülen, Farbstoffen, Radiomarkern, Polyethylenglykol oder Wirkstoffen.

### Aufgabe

Aufgabe der Erfindung ist es, Verbindungen bereitzustellen, die eine beständige Funktionalisierung und eine hohe definierte Beladung von Oberflächen erlauben, sowie Verfahren zur Herstellung dieser Verbindungen.

### Lösung der Aufgabe

Die Aufgabe der Bereitstellung von Verbindungen, die eine beständige Funktionalisierung und eine hohe definierte Beladung von Oberflächen erlauben, wird erfindungsgemäß gelöst durch Verbindungen gemäß Formel (I): 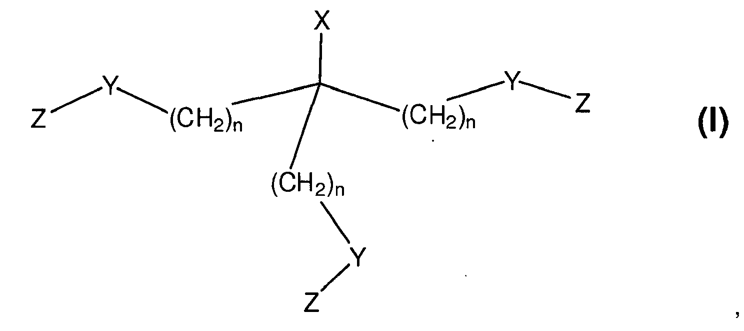 worin
- n für eine ganze Zahl zwischen 0 und 10 steht,
- Y ausgewählt ist aus -CH₂-, -CH=CH-, -C≡C-, -O-, -S-, -S-S-, -NH-, -O-NH-, -NH-O-, -HC=N-O-, -O-N=CH-, -NR¹- -Aryl-, -Heteroaryl-, -(C=O)-, -O-(C=O)-, -(C=O)-O-, -NH-(C=O)-, -(C=O)-NH-, -NR¹-(C=O)-, -(C=O)-NR¹ -NH-(C=O)-NH-, -NH-(C=S)-NH-, wobei R¹ für eine lineare Alkylgruppe mit 1 bis 10 C-Atomen oder eine verzweigte oder cyclische Alkylgruppe mit 3 bis 10 C-Atomen steht,
- Z ausgewählt ist aus 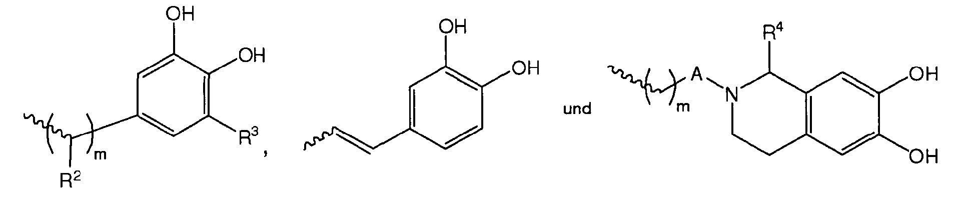 mit
   m = eine ganze Zahl zwischen 0 und 10,
   R² = -H, -OH oder -COOH,
   R³ = -H, -OH
   A = kein Atom oder -(C=O)-
   R⁴ -H oder 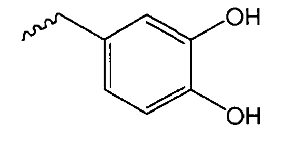 und
- X für eine -(CH₂)ₚ-R⁵ steht, worin p = 0-10 ist und R⁵ ausgewählt ist aus -H, -NH₂, -OH, -SH, -NO₂, -O-NH₂, -NH-NH₂, -N=CS, -N=C=O, -CH=CH₂, -C=CH, -COOH, -(C=O)H, -(C=O)R⁶, wobei die Hydroxy-, Thio-, Amino- oder C=O-Gruppen optional durch eine Schutzgruppe geschützt sein können, -N₃, -OR⁶, -COOR⁶, -NHR⁶, -NR⁶R⁷, -CO-NHR⁶, -CONR⁶R⁷, -NH-CO-R⁶, 4-(2,5-Dioxopyrrol-1-yl), wobei R⁶ und R⁷ unabhängig voneinander für eine lineare Alkylgruppe mit 1 bis 10 C-Atomen, eine lineare Alkenyl- oder Alkinylgruppe mit 2 bis 10 C-Atomen, eine verzweigte Alkyl-, Alkenyl- oder Alkinylgruppe mit 3 bis 10 C-Atomen oder eine cyclische Alkyl- oder Alkenylgruppe mit 3 bis 10 C-Atomen stehen, oder
   X für eine verzweigte Alkyl-, Alkenyl- oder Alkinylgruppe mit 3 bis 10 C-Atomen oder eine cyclische Alkyl-, Alkenyl- oder Alkinylgruppe mit 3 bis 10 C-Atomen oder für eine Aryl- oder Heteroarylgruppe steht,
   wobei für den Fall, dass X eine verzweigte Alkyl-, Alkenyl- und Alkinylgruppe, eine cyclische Alkyl- oder Alkenylgruppe, eine Aryl- oder Heteroarylgruppe ist, optional ein C-Atom dieser Gruppe X eine Gruppe R⁵ gemäß obiger Definition tragen kann.

Die erfindungsgemäßen Verbindungen, das Verfahren zu ihrer Herstellung sowie die Verwendung dieser Verbindungen sind nachfolgend erläutert.

Die Erfindung ist nicht auf eine der nachfolgend beschriebenen Ausführungsformen beschränkt, sondern in vielfältiger Weise abwandelbar.

Sämtliche aus den Ansprüchen, der Beschreibung und den Zeichnungen hervorgehenden Merkmale und Vorteile, einschließlich konstruktiver Einzelheiten, räumlicher Anordnungen und Verfahrensschritten, können sowohl für sich als auch in den verschiedensten Kombinationen erfindungswesentlich sein.

Die erfindungsgemäßen Verbindungen gemäß Formel (I) erlauben eine beständige Funktionalisierung und eine hohe definierte Beladung von Oberflächen. Oberflächen, die funktionalisiert und beladen werden können, umfassen Metalle, Metalloxide, Apatit, Glas und Gemische davon. Der Begriff "Apatit" umfasst dabei sowohl Verbindungen der allgemeinen Formel Ca₅(PO₄)₃(F,Cl,OH), bei denen die Konzentration von Fluorid-, Chlorid- und Hydroxylionen frei austauschbar ist, als auch die Einzelminerale Fluorapatit, Chlorapatit und Hydroxylapatit.
Unter "hoher definierter Beladung" wird verstanden, dass die Beladung der Oberfläche eine lückenlose Beschichtung des Materials in Form einer Monolage ermöglicht. Unter "Monolage" wird dabei eine Schicht von erfindungsgemäßen Molekülen auf der Oberfläche verstanden, bei der die Schichthöhe nur ein Molekül beträgt. Eine "Funktionalisierung" ist die Hinzufügung funktioneller Gruppen auf die Oberfläche eines Materials durch chemische Synthesemethoden. Eine Beschichtung von Oberflächen mit den erfindungsgemäßen Verbindungen stellt daher eine Funktionalisierung dieser Oberflächen dar. An die Gruppe X kann optional ein Effektormolekül gebunden werden, was eine weitere Funktionalisierung darstellt. Ein Effektor ist ein Molekül oder ein Molekülbestandteil, welcher einen physikalischen, chemischen, biochemischen oder biologischen Prozess auslöst oder eine solche Wirkung kontrolliert, aktiviert oder inaktiviert. Beispielse für Effektoren sind Farbstoffe, radioaktive Moleküle, Biomoleküle wie Aminosäuren, Zucker, Peptide, Proteine, DNA, RNA, Polymere wie Ethylenglycol und Derivate davon sowie Wirkstoffe. Als Wirkstoffe werden Substanzen bezeichnet, die in geringer Dosis in einem Organismus eine spezifische Wirkung, eine Reaktion, hervorrufen.
Aufgrund der multivalenten Bindung der Verbindungen gemäß Formel (I) ist diese Funktionalisierung beständig gegenüber molekularen Austauschprozessen auf der Oberfläche (wie z.B. der Hydrolyse der Verknüpfung in wässrigen Medien) und auch gegenüber mechanischer Beanspruchung.

Im Rahmen der vorliegenden Erfindung werden Alkylgruppen mit 1 bis 10 Kohlenstoffatomen ausgewählt aus Methyl, Ethyl, n-Propyl, Isopropyl, 1-Butyl, 2-Butyl, tert.-Butyl, 1-Pentyl, 2-Pentyl, 3-Pentyl, 3-Methylbutyl, 2,2-Dimethylpropyl sowie allen Isomeren von Hexyl, Heptyl, Octyl, Nonyl und Decyl. Alkenyl- und Alkinyl-gruppen umfassen mindestens zwei Kohlenstoffatome. Sie werden ausgewählt aus Ethenyl- und Ethinylgruppen sowie allen Isomeren von Propenyl-, Butenyl-, Pentenyl-, Hexenyl-, Heptenyl-, Octenyl-, Nonenyl-, Decenyl-, Ethinyl-, Propinyl-, Butinyl-, Pentinyl-, Hexinyl-, Heptinyl-, Octinyl-, Noninyl- und Decinylgruppen. Verzweigte Alkyl-, Alkenyl- und Alkinylgruppen enthalten mindestens drei Kohlenstoffatome und werden erfindungsgemäß aus den oben genannten Homologen mit dieser Mindestanzahl an Kohlenstoffatomen ausgewählt.

Dem Fachmann ist bekannt, dass cyclische Alkyl- und Alkenylgruppen mindestens drei Kohlenstoffatome enthalten müssen. Im Rahmen der vorliegenden Erfindung werden unter "ringförmigen" Gruppen solche Gruppen verstanden, bei denen alle Kohlenstoffatome an der Ringbildung beteiligt sind. "Cyclische" Gruppen können darüber hinaus noch acyclische Kohlenstoffatome enthalten. Ringförmige Alkyl-und Alkenylgruppen im Rahmen der vorliegenden Erfindung sind Propyl-, Butyl-, Pentyl-, Hexyl-, Heptyl-, Octyl-, Nonyl-, Decyl-, Propenyl-, Butenyl-, Pentenyl-, Hexenyl-, Heptenyl-, Octenyl-, Nonenyl- und Decenyl-Ringe. Handelt es sich bei den Gruppen X, R⁶ und/oder R⁷ um cyclische Alkyl- oder Alkenylgruppen, so werden diese ausgewählt aus den genannten ringförmigen Alkyl- und Alkenylgruppen, die keine weiteren Substituenten tragen, und aus den genannten ringförmigen Alkyl-und Alkenylgruppen, welche ihrerseits an eine oder mehrere acyclische Alkyl-, Alkenyl- oder Alkinylgruppen gebunden sind. Im letztgenannten Fall kann die Bindung der cyclischen Alkyl- oder Alkenylgruppe an das C1-Atom des Adamantangerüstes (falls die cyclische Gruppe X darstellt) bzw. an das entsprechende Atom der Gruppe R⁵ (falls die cyclische Gruppe R⁶ oder R⁷ darstellt) über ein cyclisches oder ein acyclisches Kohlenstoffatom der cyclischen Alkyl- oder Alkylengruppe erfolgen. Cyclische Alkylgruppen enthalten gemäß obiger Definition des Begriffes "Alkylgruppe" ebenfalls insgesamt maximal 10 Kohlenstoffatome.

Die Gruppe X ist erfindungsgemäß eine Gruppe -(CH₂)ₚ-R⁵. Handelt es sich bei R⁵ um -NH₂, -OH, -SH, -O-NH₂, -NH-NH-COOH, -(C=O)H, -(C=O)R⁶, so können diese Gruppen optional durch eine Schutzgruppe geschützt sein. Schutzgruppen für Hydroxy-, Thiol-, Amino-, Carbonyl- und Carboxylgruppen sind dem Fachmann bekannt. Er kann diese Schutzgruppen verwenden, d.h. sie einführen und bei Bedarf wieder abspalten, ohne den Schutzbereich der Patentansprüche zu verlassen.
Beispielsweise, aber nicht erschöpfend, seien folgende Schutzgruppen genannt:
- für die OH-Gruppe: Methoxymethylether (MOM), β -Methoxyethoxymethylether (MEM), Silylether, 2-Tetrahydropyranyl (THP), Acetyl (Ac), Benzoyl (Bz), Benzyl (Bn, Bnl), Dimethoxytrityl (DMT), Methoxytrityl (MMT), p-Methoxybenzylether (PMB), Methylthiomethylether, Pivaloyl (Piv), Methylether, Ethoxyethylether (EE)
- für die SH-Gruppe: tert.-Butyl, 2-Tetrahydropyranyl, Acetyl, 2-Nitropyranyl, Phenacyl, (Cumarin-4-yl)methyl
- für die NH₂-Gruppe: 1-(1-Adamantyl)-1-methoxycarbonyl (ADPOC), Allyl-oxycarbonyl (ALLOC), Benzyloxycarbonyl (Z oder Cbz abgekürzt), 9-Fluorenylmethoxycarbonyl (FMOC), p-Methoxybenzylcarbonyl (Moz, MeOZ), tert.-Butyloxycarbonyl (BOC), Acetyl (Ac), Benzoyl (Bz), Benzyl (Bn, Bnl), p-Methoxybenzyl (PMB), 3,4-Dimethoxybenzyl (DMPM), p-Methoxyphenyl (PMP), Tosyl (Ts), Sulfonamide
- für die Carbonylgruppe (in Aldehyden und Ketonen): die Umsetzung mit Diolen zu Acetalen bzw. Ketalen
- für die COOH-Gruppe: Methylester, Benzylester, tert.-Butylester, Silylester, Orthoester, Oxazoline

Unter Arylgruppen werden erfindungsgemäß Phenyl-, Naphthyl- und Anthracenylgruppen verstanden.
Heteroarylgruppe werden ausgewählt aus Furanyl, Pyrrolyl, Thiophenyl, Imidazolyl, Pyrazolyl, Triazolyl, Thiazolyl, Oxazolyl, Isooxazolyl, einem Oxadiazolyl, Pyridinyl, Pyrazinyl, Pyrimidinyl, Pyridazinyl, einem Triazinyl, einem Tetrazinyl, 1,4-Dioxinyl, einem Thiazinyl, einem Oxazinyl, einem Azepinyl, einem Diazepinyl, Benzofuranyl, Isobenzofuranyl, Indolyl, Isoindolyl, Benzothiophenyl, Benzo[c]thiophenyl, Benzimidazolyl, Purinyl, Indazolyl, Benzoxazolyl, Benzisoxazolyl, Benzothiazolyl, Chinolinyl, Isochinolinyl, Chinoxalinyl, Acridinyl, Chinazolinyl und Cinnolinyl.
Steht Y für "-Aryl-" oder "-Heteroaryl-", so sind zwei Kohlenstoffatome dieser Aryl- bzw. Heteroarylgruppe mit den Alkylengruppen -(CH₂)ₙ bzw. -(CH₂)ₘ gemäß Formel (I) und Definition von Z verbunden.

In einer vorteilhaften Ausführungsform ist Y ausgewählt aus keinem Atom, -CH₂-, -NH-(C=O)-, -(C=O)-NH-, -NR¹-, wobei R¹ wie oben definiert ist.

In einer weiteren vorteilhaften Ausführungsform ist n eine ganze Zahl zwischen 0 und 3.
In einer weiteren vorteilhaften Ausführungsform ist m eine ganze Zahl zwischen 0 und 3.
Besonders vorteilhaft stehen n und m unabhängig voneinander für ganze Zahlen zwischen 0 und 3.

In einer weiteren vorteilhaften Ausführungsform ist Z eine Gruppe 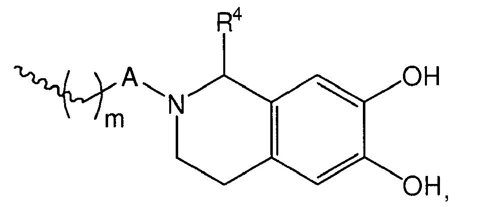 worin m, A und R⁴ wie oben definiert sind. Vorteilhaft ist m dabei eine ganze Zahl zwischen 0 und 3 ist.

In einer besonders vorteilhaften Ausführungsform ist die Gruppierung YZ ausgewählt aus

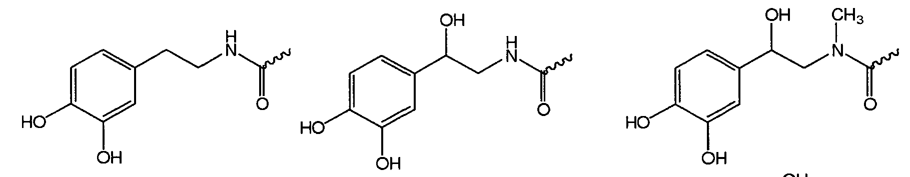

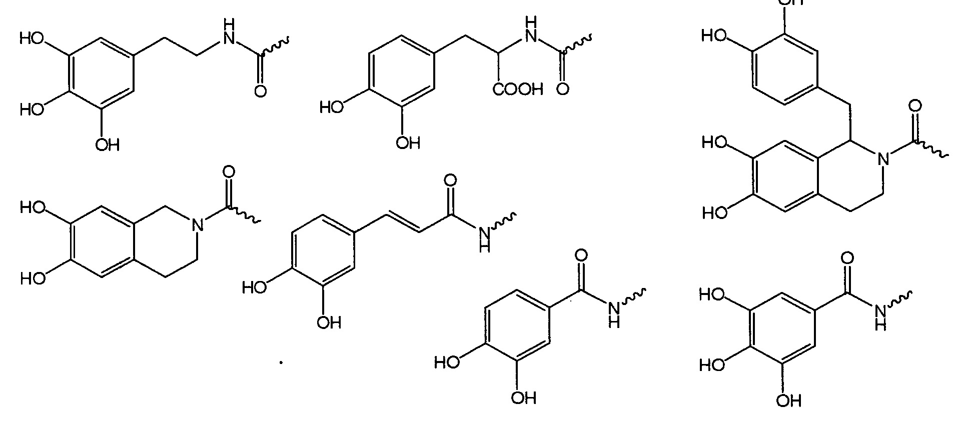

Diese besonders vorteilhaften Gruppierungen YZ leiten sich von folgenden Catecholderivaten ab:

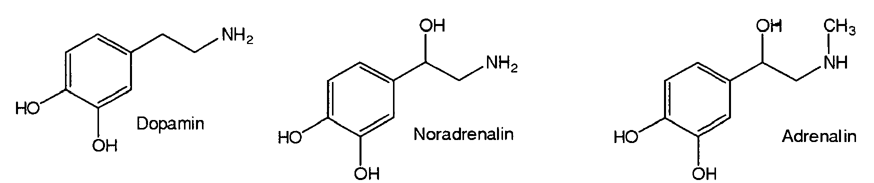

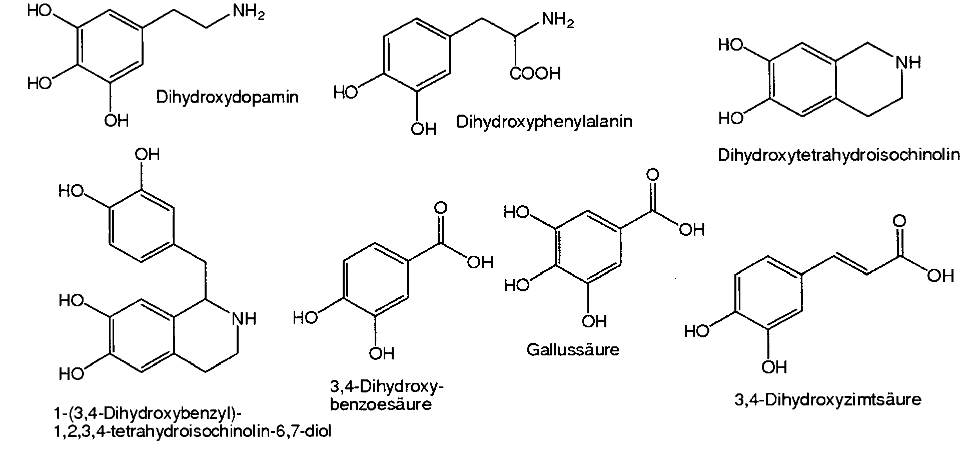

Im Rahmen der vorliegenden Erfindung werden die Verbindungen Dopamin, Noradrenalin, Adrenalin, Dihydroxydopamin, Dihydroxyphenylalanin, 1-(3,4-Dihydroxybenzyl)-1,2,3,4-tetrahydroisochinolin-6,7-diol und Dihydroxytetraisochinolin und 3,4-Dihydroxybenzoesäure als "Catecholderivate" bezeichnet.

In einer weiteren vorteilhaften Ausführungsform stellt X eine Gruppe (-CH₂)ₚ-R⁵ dar, wobei p eine ganze Zahl zwischen 0 und 3 darstellt und R⁵ wie in Anspruch 1 definiert ist.

In einer weiteren vorteilhaften Ausführungsform steht X für -(CH₂)ₚ-R⁵, worin R⁵ ausgewählt ist aus ―H, -OH, -NH₂, -NO₂, -NH-NH₂, -NHR⁶, -NR⁶R⁷, -O-NH₂, -NH-(C=O)-C≡CH, -C≡CH, -N=C=S, -N=C=O, -COOH, -(C=O)H, -(C=O)R⁶ und wobei p eine ganze Zahl zwischen 0 und 3 darstellt und R⁶ und R⁷ wie oben definiert sind.

Die erfindungsgemäßen Verbindungen gemäß Formel (I) werden hergestellt, indem eine Verbindung X-C[(CH₂)ₙ-Y']₃ mit einem Reagenz Y"Z zur korrespondierenden Verbindung X-C[(CH₂)ₙ-YZ]₃ umgesetzt und das Reaktionsprodukt anschließend gereinigt wird, worin C für das tertiäre aliphatische Kohlenstoffatom und Y' für einen Precursor (Vorläufer) der Gruppe Y gemäß Formel (I) stehen und wobei X, Z und n wie in Formel (I) definiert sind.

Unter Precursor (Vorläufer) wird hierbei eine funktionelle Gruppe verstanden, die durch Reaktion mit einer weiteren als Precursor fungierenden funktionellen Gruppe oder einem weiteren als Precursor funktionierenden Reagenz in eine funktionelle Gruppe gemäß Formel (I) umgewandelt wird.

Verbindungen der Formel X-C[(CH₂)ₙ-Y']₃ sind bekannt. Der Fachmann kann sie entweder kommerziell erwerben oder mit Hilfe seines Fachwissen nach bekannten Synthesevorschriften selbst herstellen.

In einer vorteilhaften Ausführungsform ist X ein Wasserstoffatom.

In einer weiteren vorteilhaften Ausführungsform ist X eine Gruppe -(CH₂)p-R⁵, worin p eine ganze Zahl zwischen 0 und 3 darstellt und R⁵ ausgewählt ist aus -OH, -NH₂, -NO₂, -NH-NH₂, -NHR⁶, -NR⁶R⁷ -O-NH₂, -NH-(C=O)-C≡CH, -C≡CH, - N=C=S, -N=C=O, -COOH, -(C=O)H, -(C=O)R⁶, wobei R⁶ und R⁷ wie in Formel (I) definiert sind. Diese Gruppen können, wie bereits ausgeführt, optional durch eine Schutzgruppe (Pg) geschützt sein. Werden diese Gruppen geschützt, so geschieht dies vor der Umsetzung mit dem Reagenz Y"Z, so dass in diesem Fall Pg-X-C[(CH₂)ₙ-Y']₃ mit einem Reagenz Y"Z zur korrespondierenden Verbindung Pg-X-C[(CH₂)ₙ-YZ]₃ umgesetzt wird.
Geeignete Schutzgruppen sind oben beschrieben. Dem Fachmann ist bekannt, wie man diese Schutzgruppen einführt und auch wieder entfernt. Er kann dieses Wissen anwenden, ohne den Schutzbereich der Patentansprüche zu verlassen.

Die Reinigung des Reaktionsproduktes erfolgt beispielsweise durch Entfernen des Lösungsmittels, Aufnehmen des Rückstandes in einer Mischung aus einem polar aprotischen Lösungsmittel wie Ethylacetat und einer verdünnten Mineralsäure, z.B. verdünnter Salzsäure, Waschen mit einer gesättigten KHSO₄-Lösung und Trockung.

In einer vorteilhaften Ausführungsform ist Y"Z ein Catecholamin, ausgewählt aus Dopamin, Noradrenalin, Adrenalin, Dihydroxydopamin, Dihydroxyphenylalanin, 1-(3,4-Dihydroxybenzyl)-1,2,3,4-tetrahydroisochinolin-6,7-diol und Dihydroxytetraisochinolin. In diesem Fall wird X-C[(CH₂)ₙ-Y']₃ oder Pg-X-C[(CH₂)ₙ-YZ]₃ mit dem Catecholamin in Gegenwart eines Aktivierungsreagenzes und eines Kupplungsadditivs umgesetzt. Dabei ist Y' ein Carbonsäurerest oder ein Derivat derselben. Geeignete Aktivierungsreagenzien sind beispielsweise EDC, DCC, DCI, PyClop, HBTU, HATU, HOSu, TBTU, T3P, BopCl und 3-Cl-1-Pyridiniumiodid. Als Kupplungsadditive sind beispielsweise die dem Fachmann bekannten Substanzen HOBT, HOAt, HONB und NHS verwendbar. Dem Fachmann ist bekannt, dass diese Reaktionen zweckmäßig unter Zugabe einer Base wie beispielsweise DIPEA durchgeführt werden. Dem Fachmann sind weiterhin verschiedene Lösungsmittel zur Verwendung in den genannten Verfahren bekannt. Er kann diese Kombinationen von Aktivierungsreagenzien, Kupplungsadditiven, Basen und Lösungsmitteln mit seinem üblichen Wissen und der Standardliteratur selbst herstellen.

Handelt es sich bei dem Catecholamin Y'Z um Adrenalin oder Noradrenalin, so wird deren aliphatische Hydroxygruppe optional vor der Kupplung mit einer Schutzgruppe geschützt. Ebenso kann die Carboxylgruppe des Dihydroxyphenylalanins vor der Kupplung geschützt werden, falls es Y"Z darstellt.

Wurde eine Schutzgruppe Pg eingeführt und/oder geschütztes Adrenalin, Noradrenalin oder Dihydroxyphenylalanin verwendet, so werden diese Schutzgruppen zuletzt entfernt und das entschützte Produkt anschließend gereinigt.

In einer weiteren vorteilhaften Ausführungsform ist Y"Z 3,4-Dihydroxybenzoesäure oder ein ähnliches Derivat wie beispielsweise 3,4-Dihydroxyzimtsäure oder Gallussäure. X-C[(CH₂)ₙ-Y']₃ oder Pg-X-C[(CH₂)ₙ-Y']₃ werden mit 3,4-Dihydroxybenzoesäure, 3,4-Dihydroxyzimtsäure oder Gallussäure in Gegenwart eines Aktivierungsreagenzes und eines Kupplungsadditivs umgesetzt. Y' ist hierbei vorteilhaft eine Alkohol- oder Aminfunktion. Geeignete Aktivierungsreagenzien sind beispielsweise EDC, DCC, DCI, PyClop, HBTU, HATU, HOSu, TBTU, T3P, BopCl und 3-Cl-1-Pyridiniumiodid. Als Kupplungsadditive sind beispielsweise die dem Fachmann bekannten Substanzen HOBT, HOAt und HONB verwendbar. Dem Fachmann ist bekannt, dass diese Reaktionen zweckmäßig unter Zugabe einer Base wie beispielsweise DIPEA durchgeführt werden. Dem Fachmann sind weiterhin verschiedene Lösungsmittel zur Verwendung in den genannten Verfahren bekannt. Er kann diese Kombinationen von Aktivierungsreagenzien, Kupplungsadditiven, Basen und Lösungsmitteln mit seinem üblichen Wissen und der Standardliteratur selbst herstellen.

In einer weiteren vorteilhaften Ausführungsform ist Y"Z ein Catecholamin, ausgewählt aus Dopamin, Noradrenalin, Adrenalin, Dihydroxydopamin, Dihydroxyphenylalanin, 1-(3,4-Dihydroxybenzyl)-1,2,3,4-tetrahydroisochinolin-6,7-diol und Dihydroxytetraisochinolin. X-C[(CH₂)ₙ-Y']₃ oder Prot-X-C[(CH₂)ₙ-Y']₃ werden mit dem Catecholamin in Gegenwart eines Reduktionsmittels umgesetzt. Y' ist dabei ein Aldehyd oder ein Keton. Geeignete Reduktionsmittel sind beispielsweise NaBH₄, NaBH₃CN, NaBH(OAc)₃ sowie H₂ und Metallkatalysatoren. Dem Fachmann sind verschiedene Lösungsmittel zur Verwendung in den genannten Verfahren bekannt. Er kann diese Kombinationen von Reduktionsmitteln und Lösungsmitteln mit seinem üblichen Wissen und der Standardliteratur selbst herstellen.

In einer weiteren vorteilhaften Ausführungsform ist Y"Z ein Catecholamin, ausgewählt aus Dopamin, Noradrenalin, Adrenalin, Dihydroxydopamin, Dihydroxyphenylalanin, 1-(3,4-Dihydroxybenzyl)-1,2,3,4-tetrahydroisochinolin-6,7-diol und Dihydroxytetraisochinolin. X-C[(CH₂)ₙ-Y']₃ oder Prot-X-C[(CH₂)ₙ-Y']₃ mit einer geeigneten Austrittsgruppe Y' werden mit dem Catecholamin umgesetzt. Geeignete Austrittsgruppen sind beispielsweise -OTs, OMs, -OTf sowie Halogenide. Dem Fachmann sind verschiedene Lösungsmittel zur Verwendung in den genannten Verfahren bekannt. Er kann diese Kombinationen von Austrittsgruppen und Lösungsmitteln mit seinem üblichen Wissen und der Standardliteratur selbst herstellen.

In einer weiteren vorteilhaften Ausführungsform ist Y"Z ein Catecholamin, ausgewählt aus Dopamin, Noradrenalin, Adrenalin, Dihydroxydopamin, Dihydroxyphenylalanin, 1-(3,4-Dihydroxybenzyl)-1,2,3,4-tetrahydroisochinolin-6,7-diol und Dihydroxytetraisochinolin. X-C[(CH₂)ₙ-Y']₃ oder Pg-X-C[(CH₂)ₙ-Y']₃ werden mit dem Catecholamin umgesetzt. Y' ist dabei ein Isothiocyanat oder ein Isocyanat. Dem Fachmann sind verschiedene Lösungsmittel zur Verwendung in den genannten Verfahren bekannt. Er kann diese Kombinationen von Reduktionsmitteln und Lösungsmitteln mit seinem üblichen Wissen und der Standardliteratur selbst herstellen.

In einer weiteren vorteilhaften Ausführungsform ist Y"Z 3,4-Dihydroxybenzaldehyd. X-C[(CH₂)ₙ-Y']₃ oder Pg-X-C[(CH₂)ₙ-Y']₃ werden mit 3,4-Dihydroxybenzaldehyd umgesetzt. Y' ist dabei ein O-Alkylhydroxylamin oder das korrespondierende Hydrohalogenid. Dem Fachmann sind verschiedene Lösungsmittel zur Verwendung in den genannten Verfahren bekannt. Er kann diese Kombinationen von Reduktionsmitteln und Lösungsmitteln mit seinem üblichen Wissen und der Standardliteratur selbst herstellen.

In einer weiteren vorteilhaften Ausführungsform ist Y"Z 3,4-Dihydroxybenzoesäure oder ein ähnliches Derivat wie beispielsweise 3,4-Dihydroxyzimtsäure oder Gallussäure. X-C[(CH₂)ₙ-Y']₃ oder Pg-X-C[(CH₂)ₙ-Y']₃ werden mit 3,4-Dihydroxybenzoesäure oder einem ähnlichen Derivat wie beispielsweise 3,4-Dihydroxyzimtsäure oder Gallussäure in Gegenwart eines Aktivierungsreagenzes wie beispielsweise DCC oder EDC oder mit katalytischen Mengen einer Säure wie beispielsweise HCl, H₂SO₄ oder p-Toluolsulfonsäure umgesetzt. Y' ist hierbei vorteilhaft eine Alkoholfunktion. Dem Fachmann sind verschiedene Lösungsmittel zur Verwendung in den genannten Verfahren bekannt. Er kann diese Kombinationen von Aktivierungsreagenzien, Kupplungsadditiven, Basen und Lösungsmitteln mit seinem üblichen Wissen und der Standardliteratur selbst herstellen.

Die erfindungsgemäßen Verbindungen gemäß Formel (I) können in einem Verfahren zur Funktionalisierung von Oberflächen verwendet werden. Die Funktionalisierung geschieht dabei mittels dip and rinse, indem die zu funktionalisierenden Oberflächen in eine Lösung der erfindungsgemäßen Verbindungen getaucht werden.

Vorteilhaft werden die erfindungsgemäßen Verbindungen in einer wässrigen Pufferlösung gelöst, die eine Salzkonzentration aufweist, welche signifikant höher als physiologische Salzkonzentration (0,9 Gew.-% NaCl) ist. Ein geeigneter Puffer ist beispielsweise MOPS (3(N-Morpholin)-propansulfonsäure), geeignete Salze sind beispielsweise NaCl und K₂SO₄ und Gemische davon. Die Salzkonzentration beträgt vorteilhaft zwischen 10 und 20 Gew.-% und die Pufferkonzentration 0,05 bis 0,2 mmol.

Optional kann die Gruppe X der erfindungsgemäßen Verbindungen an einen Effektor gekuppelt sein. Die Kupplung von X an den Effektor kann dabei sowohl in Lösung, d.h. vor der Oberflächenfunktionalisierung, als auch auf der Oberfläche, d.h. nach der Oberflächenfunktionalisierung, erfolgen. Effektoren sind beispielsweise Ethergruppierungen, Estergruppierungen, Heteroaromaten, Farbstoffe, Metallkomplexe, Polymere (beispielsweise Polyethylenglykole), pharmazeutische Wirkstoffe (beispielsweise Antibiotika, Bisphosphonate), Biomoleküle (z.B. eine Aminosäure), Peptide, Kohlenhydrate und Terpene. Ist der Effektor ein Polymer und handelt es sich dabei um ein Polyethylenglykol, so handelt es sich vorteilhaft um eine Gruppierung ―(O-CH₂-CH₂)_{q}-R⁵ oder -(CH₂-CH₂-O)_{q}-R⁵, worin q eine Zahl zwischen 1 und 10 und R⁵ wie unter Formel 1 beschrieben definiert ist.

In einer vorteilhaften Ausführungsform erfolgt die Kupplung von X dabei mittels Click-Chemie. Unter "Click-Reaktionen" versteht der Fachmann energetisch begünstigte Reaktionen, die spezifisch verlaufen und ein einziges Produkt ergeben. Es handelt sich damit um sehr einfach durchzuführende und effiziente Reaktionen. Click-Reaktionen finden Anwendung in Molekularbiologie, Wirkstoffentwicklung, Biotechnologie, makromolekularer Chemie und Materialwissenschaften.
Das Konzept der Click-Reaktion wurde von K. Barry Sharpless begründet und beschreibt Reaktionen, die
- modular aufgebaut sind,
- ein breites Anwendungsspektrum aufweisen,
- mit hohen Ausbeuten durchführbar sind,
- stereospezifisch ablaufen,
- einfache Reaktionsbedingungen erlauben (möglichst unempfindlich gegen Wasser und Sauerstoff),
- in umweltfreundlichen und/oder leicht entfernbaren Lösungsmitteln wie Wasser oder lösungsmittelfrei ablaufen,
- eine einfache Aufreinigung (Extraktion, Phasentrennung, Destillation oder Kristallisation) oder gar keine Aufreinigung benötigen.

"Click-Reaktionen sind in der Regel stark thermodynamisch begünstigt, häufig mehr als 84 kJ/mol, was in einem schnellen Umsatz mit hoher Selektivität für ein einzelnes Produkt resultiert. Dabei handelt es sich meist um Kohlenstoff-Heteroatom-Bindungsbildungen.

Chemische Reaktionen, die diese Kriterien erfüllen, sind:
- die Carbonylchemie des "Non-Aldol-Typs" wie z.B. die Bildung von Harnstoff, Thioharnstoff, Oximen, Iminen, aromatischen Heterocyclen und Hydrazonen sowie die Bildung von Carbamiden und Amiden,
- Cycloadditionen an ungesättigten C-C-Bindungen, besonders 1,3-dipolare Cycloadditionen wie die Huisgen-Cycloaddition, außerdem Diels-Alder-Reaktionen,
- nucleophile Substitutionen, besonders die Ringöffnung von gespannten, elektrophilen Heterozyklen wie Aziridinen und Epoxiden,
- Additionsreaktionen an C-C-Mehrfachbindungen, meist oxidativ wie z.B. Epoxidierung, Aziridierung oder Dihydroxylierung, aber auch Michaeladditionen von Nu-H, wobei Nu ein Nucleophil ist.

In einer weiteren vorteilhaften Ausführungsform erfolgt die Kupplung des Effektors an die Gruppe X über konventionelle Substitutions- oder Additionsreaktionen, die nicht die oben genannten Bedingungen einer Click-Reaktion gehören. Zu diesen konventionellen Reaktionen zählen beispielsweise die Etherbildung, die Veresterung einer Carbonsäure oder die Amidbildung.

Besonders vorteilhaft handelt es sich bei den Oberflächen, die funktionalisiert werden sollen, um metallische Oberflächen umfassend Eisen und/oder Titan oder um Oberflächen umfassend Apatit. Dem Fachmann ist bekannt, dass Knochen von Wirbeltieren zu etwa 50 % aus Apatit bestehen, Dentin zu etwa 70 % und Zahnschmelz zu mehr als 95 %. Moderner Zahnersatz, beispielsweise Füllungen und Zahnimplantate, umfasst häufig Apatit und/oder Vorrichtungen, die Eisen und/oder Titan umfassen. Es ist ferner bekannt, dass die Oberflächen von Endoprothesen, z.B. für Hüft- und Kniegelenke, Eisen und/oder Titan umfassen. Die erfindungsgemäßen Verbindungen gemäß Formel (I) sowie die daraus erhältlichen, an einen Effektor gekuppelten Verbindungen eignen sich daher zur Oberflächenfunktionalisierung von Zahnersatz und Gelenksendoprothesen.

### Ausführungsbeispiele

### Ausführungsbeispiel 1 :

### Herstellung von 4-[2-(3,4-dihydroxyphenethylaminocarbonyl)ethyl]-N1, N7-bis(dihydroxyphenethyl)-4-(propiolsäureamid)heptandiamid 5

### Syntheseplan:

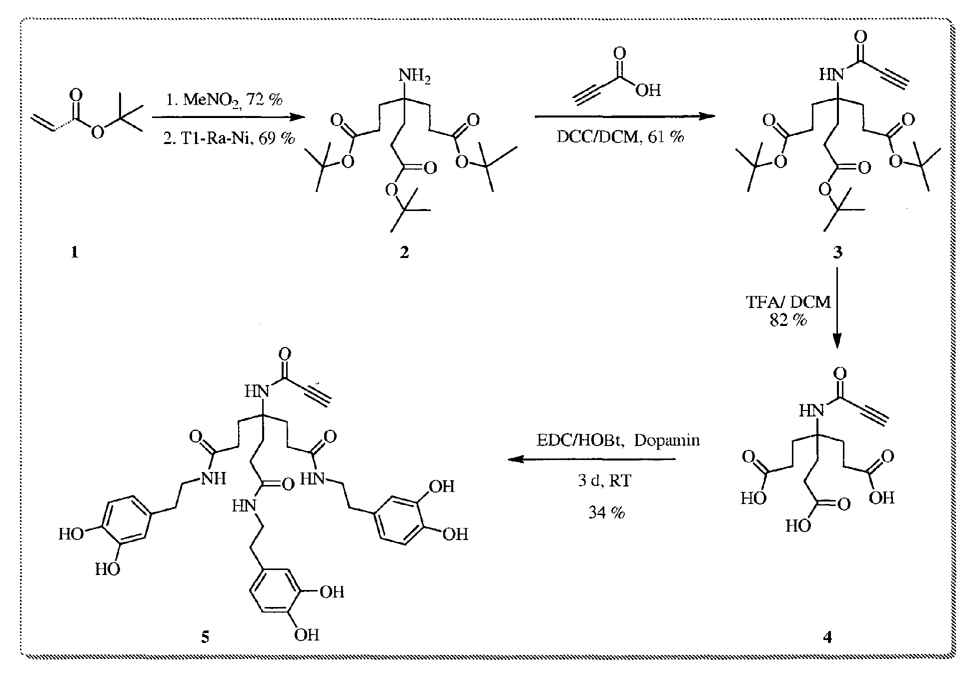

Die Tricarbonsäure **4** (0.32 g, 1.10 mmol) und EtN₃ (1.44 mL; 10.4 mmol) wurden in abs. DMF gelöst und auf 0 °C gekühlt. Nach der Zugabe von EDC (0.67 g, 4,3 mmol) und HOBt (0,58 g, 4.3 mmol) wurde das Reaktionsgemisch für 30 min gerührt. Danach wurde das Dopamin (0.65 g, 4.3 mmol) und EtN₃ (0.91 mL, 6,6 mmol) gelöst in DMF zugetropft und 3 d bei RT gerührt. Das erhaltene Rohprodukt wurde nach Säure-Base-Extraktion und Gefriertrocknung mehrmals in Et20 gerührt, um das entstandene HOBt zu entfernen. Das Triscatechol 5 (0.06 g, 0,085 mmol, 8 %) wurde als farbloser Feststoff als Produkt erhalten.

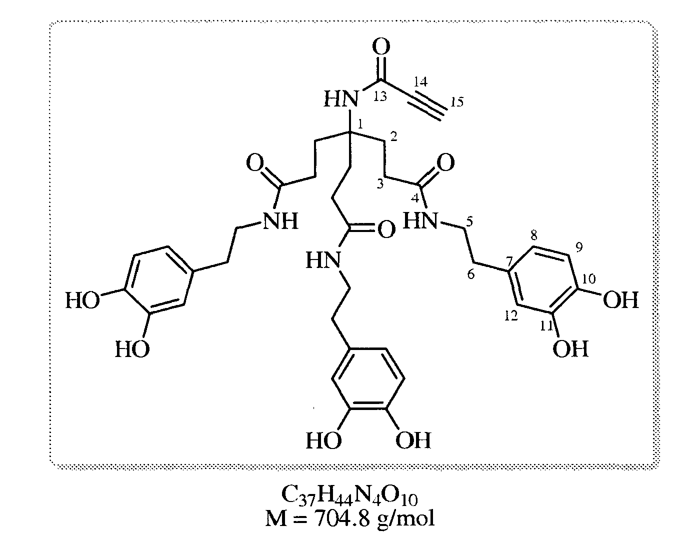

**¹H-NMR (400 MHz, MeOD):** δ [ppm] = 6,68 (d, 3H, ³*J* = 7.9 Hz, 9-H), 6,64 (s, 3H, 12-H), 6.52 (d, 3H, ³*J*= 7.9 Hz, 8-H), 3.51 (s, 1 H, 15-H), 3.34 (m, 6H, 6-H), 2.63 (t, 6H, ³*J*= 7.1 Hz 5-H), 2.12 (s, 6H, 3-H), 2.13-2.09 (m, 6H, 3-H), 1.94-1.92 (m, 6H, 2-H); **¹³C-NMR (100 MHz, MeOD):** δ [ppm] = 175.5 (C4), 146.2 (C11), 144.8 (C10), 130.2 (C12), 121.1 (C8), 117.0 (C9), 116.4 (C7), 74.6 (C15), 60,3 (C1), 42.4 (C5), 34.8 (C6), 31.5 (C3), 31.2 (C2); **HRMS** (ESI): berechnet für C₃₇H₄₄N₄O₁₀ [M+Na⁺] = 727.2955, gefunden: 727.2961.

### Ausführungsbeispiel 2:

### 4-[2-(3,4-dihydroxyphenethylaminocarbonyl)ethyl]-N1,N7-bis(dihydroxyphenethyl)-4-aminoheptandiamid 10

### Syntheseplan:

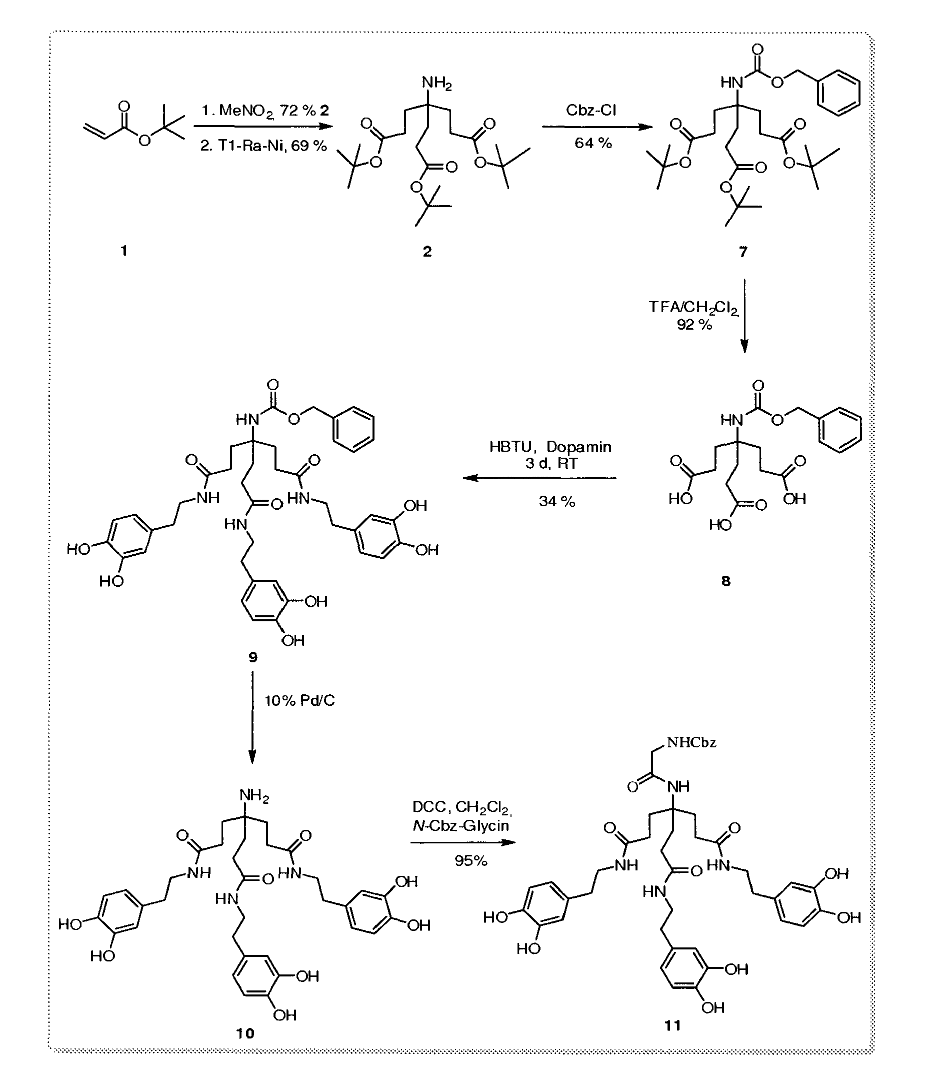

### a) Di-tert-Butyl-4-benzyloxycarbonyl-4-[2-(tert-butoxycarbonyl)ethyl]-heptandioat 7

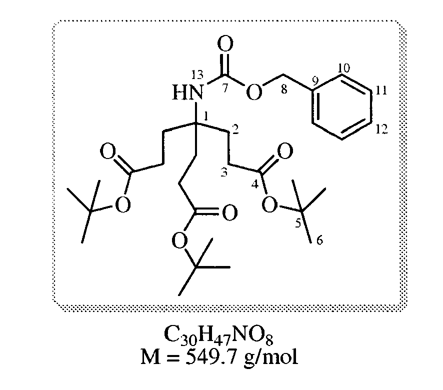

Das Amin 2 (3.0 g, 7.2 mmol) wurde in Dioxan/H₂O (100 mL; 1:1) bei RT gelöst, auf 0 °C gekühlt und mit NaHCO₃ (0.7 g, 8.3 mmol) versetzt. Anschließend wurde bei 0 °C Cbz-Cl (1.4 g, 1.2 mL, 8.3 mmol) über 10 min zugetropft. Die Reaktionslösung wurde 2 h bei 0 °C und anschließend 2 h bei RT gerührt. Es wurde viermal mit CH₂Cl₂ extrahiert, die vereinigten organischen Phasen mit wässriger 1 N HCl gewaschen und über Na₂SO₄ getrocknet. Nach Abziehen des Lösungsmittels im Vakuum wurde das Rohprodukt säulenchromatographisch gereinigt. Als Produkt wurde ein farbloses Feststoff des Cbz-geschützten Amins 7 (2.5 g, 4.6 mmol, 64 %) erhalten.

**Smp.:** 100 °C; ***R*_{f}** = 0.42 (PE/ EtOAc; 8.5:1.5); Cersulfat; **¹H-NMR (400 MHz, CDCl₃):** δ [ppm] = 7.37-7.30 (m, 5 H, 10-H, 11-H, 12-H), 5.04 (s, 2H, 8-H), 4.83 (s, 1 H, 13-H), 2.20 (t, 6H, ³*J* = 7.52 Hz, ³*J* = 8.26 Hz, 3-H), 1.91 (t, 6H, ³*J* = 8.24 Hz, ³ *J* = 7.43 Hz, 2-H), 1.43 (s, 27H, 6-H); **¹³C-NMR (100 MHz, CDCl₃):** δ [ppm] = 172.7 (C4), 154.3 (C7), 136.7 (C9), 128.6 (C10), 128.2 (C11), 128.1 (C12), 80.7 (C5), 66.3 (C8), 56.7 (C1), 30.2 (C3), 29.8 (C2), 28.2 (C6); **IR (KBr-Pressling):** v [cm⁻¹] = 3348, 1726, 757; **HRMS** (ESI): berechnet für C₃₀H₄₇NO₈ [M+Na⁺] = 572.3199, gefunden: 572.3195; **CHN-Analyse**: gefunden (berechnet) [%]: C = 65.63 (65.55), H = 8.65 (8.62), N = 2.55 (2.55).

### b) 4-Benzyloxycarbonyl-4-(2-carboxyethyl)-heptandisäure 8

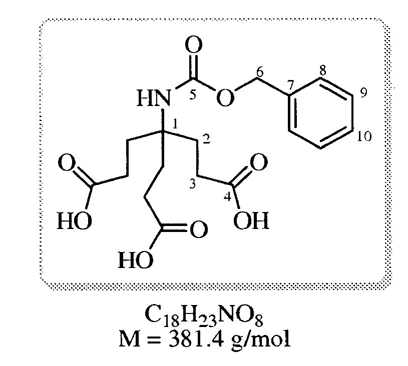

Der Triester 7 (2.0 g, 3.6 mmol) wurde in TFA (45 mL) und CH₂Cl₂ (135 mL) gelöst und 4 h bei RT gerührt. Anschließend wurde fünfmal mit CH₂Cl₂ koevaporiert und das Lösungsmittel im Vakuum abdestilliert. Der Rückstand wurde in EtOAc und wässriger 1 N HCl aufgenommen, dreimal mit wässriger 1 N HCl gewaschen und die vereinigten organischen Phasen über Na₂SO₄ getrocknet. Nach Entfernen des Lösungsmittels im Vakuum wurde die Tricarbonsäure **8** (1.3 g, 3.3 mmol, 92 %) als farbloser Feststoff erhalten.

**¹H-NMR (400 MHz, DMSO):** ö [ppm] = 7.32-7.29 (m, 5 H, 8-H, 9-H, 10-H), 4.98 (s, 2H, 6-H), 2.12 (t, 6H, ³*J*= 7.78 Hz, ³*J*= 7.83 Hz, 3-H), 1.79 (t, 6H, ³*J*= 8.38 Hz, ³*J* = 7.78 Hz, 2-H); **¹³C NMR (100 MHz, DMSO):** δ [ppm] = 174.5 (C4), 154.3 (C5), 137.5 (C7), 128.4 (C8), 127.7 (C9), 127.5 (C10), 64.7 (C6), 55.8 (C1), 29.2 (C3), 28.1 (C2); **IR (KBr-Pressling):** v [cm⁻¹] = 3433, 1712, 697; **HRMS** (ESI): berechnet für C₁₈H₂₃NO₈ [M-Na⁺] = 404.1321, gefunden: 404.1326; **CHN-Analyse:** gefunden (berechnet) [%]: C = 52.46 (56.69), H = 6.17 (6.08), N = 3.34 (3.67).

### c) 4-Benzyloxycarbonyl-4-[2-(3,4-dihydroxyphenethylaminocarbonyl)ethyl]-N1, N7-bis-(dihydroxyphenethyl)-4-heptandiamid 9

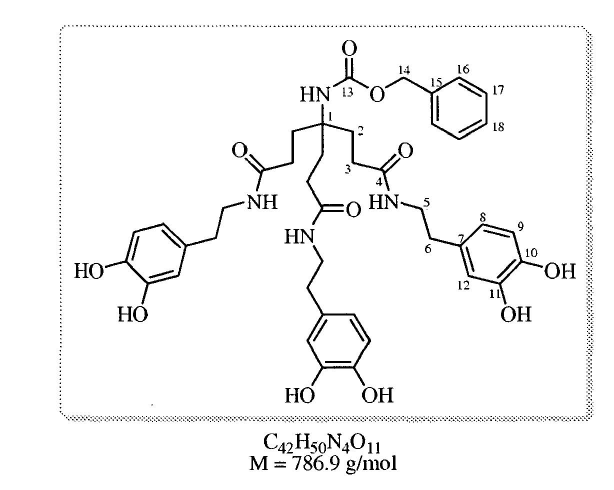

Die Tricarbonsäure **8** (0.20 g, 0.52 mmol) wurde mit Dopamin (0.26 g, 1.72 mmol) in DMF (20 mL) nach AAVX umgesetzt. Das erhaltene Rohprodukt wurde nach Gefriertrocknung dreimal in Et₂O gerührt, um das entstandene HOBt zu entfernen. Nach Abdekantieren des Et₂O wurde das gekuppelte Produkt 9 (0.14 g, 0.18 mmol, 34 %) als farbloser Feststoff erhalten.

**¹H-NMR (400 MHz, CDCl₃):** δ [ppm] = 7.35-7.30 (m, 5H, 16-H, 17-H, 18-H), 6.60 (d, 3H, ³*J*= 7.87 Hz, 9-H), 6.57 (s, 3H, 12-H), 6.42 (d, 3H, ³*J*= 7.87 Hz, 8-H), 5.00 (s, 2H, 14-H), 3.41-3.36 (m, 6H, 5-H), 3.15-3.14 (m, 6H, 6-H), 2.00 (s, 6H, 3-H), 1.78 (s, 6H, 2-H); **¹³C-NMR (100 MHz, CDCl₃):** δ [ppm] = 172.0 (C4), 145.1 (C11), 143.5 (C10), 137.4 (C15), 130.3 (C7), 128.4 (C16), 127.7 (C17), 127.5 (C18), 64.9 (C14), 56.2 (C1), 40.7 (C5), 34.8 (C3), 30.4 (C6), 29.7 (C2); **HRMS** (ESI): berechnet für C₄₂H₅₀N₄O₁₁ [M-Na⁺] = 809.3374, gefunden: 809.3374.

### d) 4-[2-(3,4-dihydroxyphenethylaminocarbonyl)ethyl]-N1,N7-bis(dihydroxyphenethyl)-4-aminoheptandiamid 10

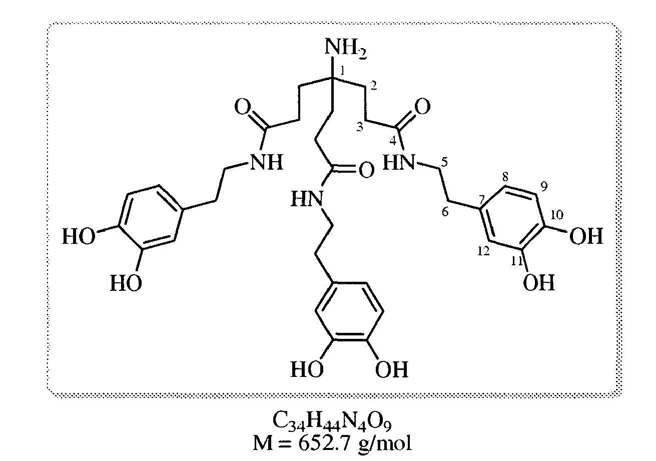

Das gekuppelte Produkt 9 (0.035 g, 0.044 mmol) wurde in abs. MeOH (10 mL) gelöst und nach AAVX umgesetzt. Nach der Filtration wurde das Amin 10 als Rohprodukt (0.035 mg) als braunes Öl erhalten.

**¹H-NMR (400 MHz, CDCl₃):** ö [ppm] = 7.35-7.30 (m, 5H, 16-H, 17-H, 18-H), 6.47 (d, 3H, ³*J*= 7.93 Hz, 9-H), 6.56 (s, 3H, 12-H), 6.42 (d, 3H, ³*J*= 7.93 Hz, 8-H), 3.17 (s, 12H, 5-H, 6-H), 2.07 (s, 6H, 3-H), 1.52 (s, 6H, **2-H); ¹³C-NMR (100** MHz, **CDCl₃):** ö [ppm] = 172.0 (C4), 145.0 (C11), 143.5 (C10), 130.2 (C7), 119.1 (C12), 115.9 (C9), 115.4 (C8), 48.6 (C1), 40.6 (C5), 34.6 (C3), 34.0 (C6), 29.7 (C2); **HRMS** (ESI): berechnet für C₃₄H₄₄N₄O₉ [M-H⁺] = 653.3187, gefunden: 653.3196.

### e) 4-Benzyloxycarbonyl-Glycin-4-[2-(3,4-dihydroxyphenethylaminocarbonyl)ethyl]-N1, N7-bis-(dihydroxyphenethyl)-4-heptandiamid 11

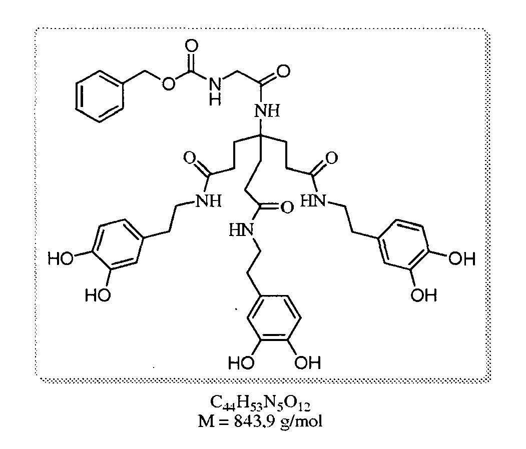

Das Amin 10 (3.8 g, 9.1 mmol) wurde in abs. CH₂Cl₂ (100 mL) bei 0 °C gelöst und mit Cbz-Glycin (2.8 g, 13.6 mmol), gelöst in abs. CH₂Cl₂ (50 mL), versetzt. Anschließend wurde DCC (2.8 g, 13.6 mmol) hinzugefügt und 24 h bei RT gerührt. Das ausgefallene DCU wurde abfiltriert und das CH₂Cl₂ im Vakuum abgezogen und der Rückstand mit EtOAc aufgenommen. Die Reaktionslösung wurde zweimal mit gesättigter, wässriger KHSO₄-Lösung und zweimal mit gesättigter, wässriger NaCl-Lösung gewaschen. Die vereinigten organischen Phasen wurden über Na₂SO₄ getrocknet und EtOAc im Vakuum abgezogen. Das Cbz-Glycin gekuppelte Triscatechol **11** (5.2 g, 8.5 mmol, 95%) wurde als farbloses Produkt erhalten.

**MS** (ESI): m/z (%) = 866.5 (58) [M+Na⁺], 844.3 (50) [M+H⁺].

### Ausführungsbeispiel 3:

### 4-[2-(3,4-dihydroxyphenethylaminocarbonyl)ethyl]-N¹, N⁷-bis(dihydroxyphenethyl)-4-nitroheptandiamid 13

### Syntheseplan:

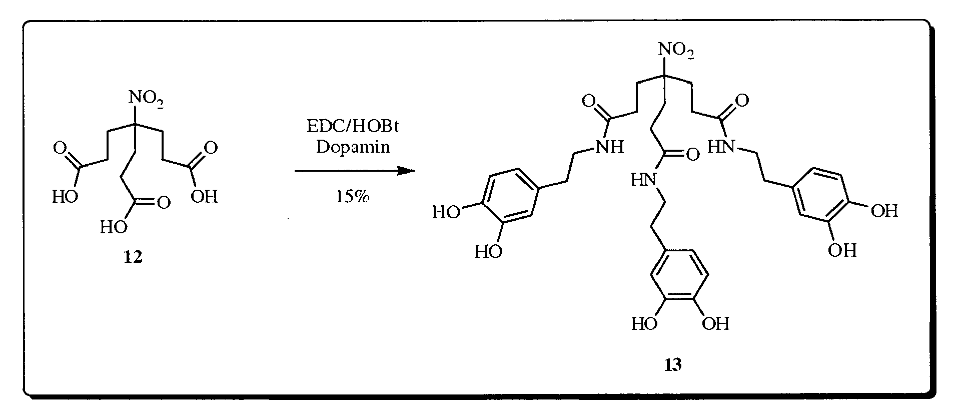

Die Tricarbonsäure **12** (0.1 g, 0.36 mmol) und EtN₃ (0.47 mL; 3.40 mmol) wurden in abs. DMF (10 mL) gelöst und auf 0 °C gekühlt. Nach der Zugabe von EDC (0.25 g, 1.62 mmol) und HOBt (0.22 g, 1.62 mmol) wurde das Reaktionsgemisch für 5 min gerührt. Danach wurde das Dopamin (0.25 g, 1.62 mmol) und EtN₃ (0.47 mL, 3.40 mmol) gelöst in DMF zugetropft und 3 d bei RT gerührt. Das DMF wurde im Vakuum entfernt und der Rückstand mit wässriger 1 N HCl-Lösung gewaschen und die organische Phase über Na₂SO₄ getrocknet. Nach Gefriertrocknung wurde das Rohprodukt mehrmals in Et₂O gerührt, um das entstandene HOBt zu entfernen. Das Triscatechol 13 (0.04 g, 0.06 mmol, 15%) wurde als farbloser Feststoff erhalten.

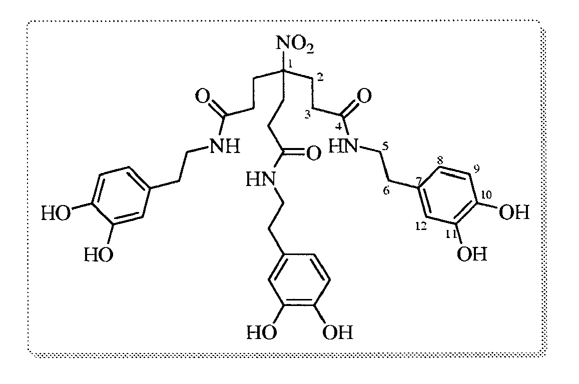

**¹H-NMR (400 MHz, CH₃OD):** ö [ppm] = 6,68 (d, 3H, ³*J*= 8.0 Hz, 9-H), 6,6 (d, 3H, ⁴*J*= 2.0 Hz 12-H), 6.50 (dd, 3H, ³*J*= 8.1 Hz, ⁴*J*= 2.0 Hz, 8-H), 3.34-3.30 (m, 6H, 6-H), 2.63 (t, 6H, ³*J*= 7.4 Hz, 5-H), 2.16-2.09 (m, 12H, 2-H, 3-H); **MS** (ESI): m/z (%) = 705.3 (85) [M+Na⁺].

### Ausführungsbeispiel 4:

### Oberflächenmodifizierung

Für die Oberflächenmodifizierung werden TiO₂-Oberflächen auf Si-Wafern verwendet. Dazu wird eine Lösung aus TiCl₄, Wasser und Ethanol hergestellt und die Si-Wafer hineingetaucht und mit einer Geschwindigkeit von 1 mm/sec herausgezogen. Im Ofen werden die Oberflächen zu Ihrer endgültigen Form "gebrannt". Hierzu werden die Si-Wafer 6 h auf 80 °C erhitzt, anschließend für 6 h auf 300 °C und dann mit einer Heizrate von 10 °C/min auf 550 °C.

Die erfindungsgemäßen Verbindungen (je 5mg) werden in 5 mL MeOH und 15 mL HPLC reinen Wassers gelöst.

Für die Dipcoating-Methode wird eine 100 mL Puffer-Stammlösung (0,1 mol/l Mops) aus 3.5 g NaCl, 10.45 g K₂SO₄ und 2.31 g Mops (= 3-Morpholinopropan-1-sulfonsäure) hergestellt. Hierbei wird ebenfalls HPLC-reines Wasser verwendet.
5 mL der Lösung jeder erfindungsgemäßen Verbindung und 10 mL Puffer-Stammlösung werden gemischt und die TiO₂ Oberflächen darin für 13 h mittels Dip-Coating beschichtet.

Nach dieser Reaktionszeit werden die Oberflächen mit dest. MeOH und dest. Wasser gereinigt, mittels Druckluft getrocknet und mittels Kontaktwinkelmessung und SIMS-Tof vermessen.

### Ausführungsbeispiel 5:

### Kontaktwinkelmessung

Vermessen wurden folgende Substanzen:

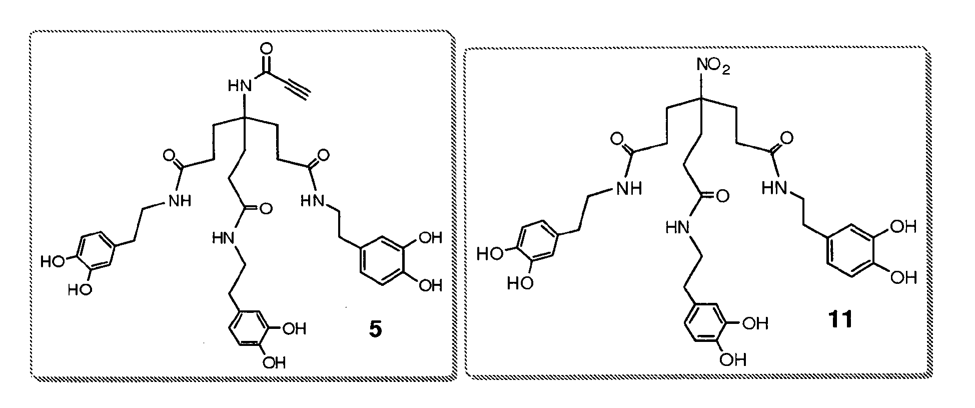

Vor jeder Messung wurden die zu vermessenden TiO₂-Oberflächen mittels dest. MeOH und Druckluft von Staub und Wassertropfen gereinigt.
Zur Messung wurde ein Kontaktwinkelmessgerät (Krüss Dropshape Analyser, DAS 10-MK 2) verwendet, wobei in der Messkammer eine konstante Temperatur von 23 ° herrschte. Eine Nadel, welche einen Wassertropfen (HPLC grade water) von 4µl herstellte, wurde langsam auf die Oberfläche gefahren um den Tropfen auf der Oberfläche absetzten zu lassen.
Ab diesem Zeitpunkt nahm eine Kamera eine 1000 Bildersequenz auf, die am Ende manuell mit Hilfe eines speziellen Computerprogrammes (Krüss, DSA = Drop Shape Analyze), welches die Young-Laplace Methode (Sessel Drop Fitting) verwendet, ausgewertet.
Jede TiO₂ Oberfläche wurde 3-4-mal vermessen und aus den Ergebnissen der Mittelwert bestimmt.

Als Kontrollen wurde jeweils das ein reines, gereinigtes TiO₂-Blech ohne Beschichtung (zweite Spalte in Tabelle 1) sowie mit Mops-Puffer beschichtetes TiO₂-Blech (Spalte 3 in Tabelle 1) vermessen. In Spalte 4 sind die Ergebnisse der Kontaktwinkelmessungen von TiO₂-Blechen gezeigt, die zuvor mit in Mops-Puffer gelösten Substanzen 5 und 11 ("Moleküle") beschichtet worden waren.

**Tab. 1: Ergebnisse der Kontaktwinkelmessung**

| | TiO₂ | TiO₂ + Mops | TiO₂ + Mops + Moleküle |
|---|---|---|---|
| Substanz Nr. | Kontaktwinkel (°) | Kontaktwinkel (°) | Kontaktwinkel (°) |
| 5 | 27,9 | 8,1 | 18,5 |
| 11 | 32,3 | 5,8 | 22,9 |

## Patentansprüche

1. Verbindungen gemäß Formel (I): 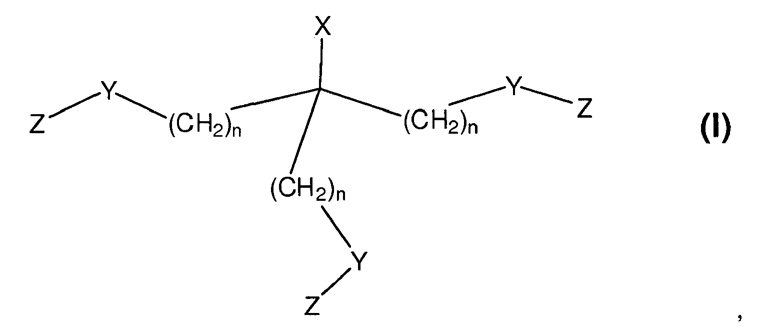 worin
- n für eine ganze Zahl zwischen 0 und 10 steht,
- Y ausgewählt ist aus -CH₂-, -CH=CH-, -C≡C-, -O-, -S-, -S-S-, -NH-, -O- -NH-, -NH-O-, -HC=N-O-, -O-N=CH-, -NR¹-, -Aryl-, -Heteroaryl-, -(C=O)-, -O-(C=O)-, -(C=O)-O-, -NH-(C=O)-, -(C=O)-NH-, -NR¹-(C=O)-, -(C=O)-NR¹-, -NH-(C=O)-NH-, -NH-(C=S)-NH-, wobei R¹ für eine lineare Alkylgruppe mit 1 bis 10 C-Atomen oder eine verzweigte oder cyclische Alkylgruppe mit 3 bis 10 C-Atomen steht,
- Z ausgewählt ist aus 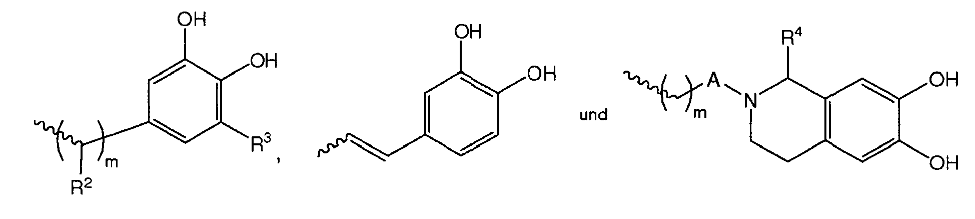 mit
m = eine ganze Zahl zwischen 0 und 10,
R² = -H, -OH oder -COOH,
R³ = -H, -OH
A = kein Atom oder -(C=O)-
R⁴ = -H oder 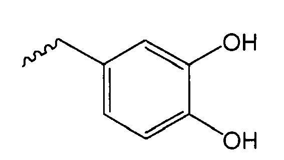 und
- X für eine Gruppe -(CH₂)ₚ-R⁵ steht, worin p = 0-10 ist und R⁵ ausgewählt ist aus -H, -NH₂, -NO₂, -OH, -SH, -O-NH₂, -NH-NH₂, -N=C=S, -NC=O, -CH=CH₂, -pech, -COOH, -(C=O)H, -(C=O)R⁶. wobei die Hydroxy-, Thio-, Amino- oder C=O-Gruppen optional durch eine Schutzgruppe geschützt sein können, -N₃, -OR⁶, -COOR⁶, -NHR⁶, -NR⁶R⁷, -CO-NHR⁶, -CONR⁶R⁷, -NH-CO-R⁶, 4-(2,5-Dioxopyrrol-1-yl), wobei R⁶ und R⁷ unabhängig voneinander für eine lineare Alkylgruppe mit 1 bis 10 C-Atomen, eine lineare Alkenyl- oder Alkinylgruppe mit 2 bis 10 C-Atomen, eine verzweigte Alkyl-, Alkenyl- oder Alkinylgruppe mit 3 bis 10 C-Atomen oder eine cyclische Alkyl- oder Alkenylgruppe mit 3 bis 10 C-Atomen stehen, oder
wobei für den Fall, dass X ein verzweigte Alkyl-, Alkenyl- und Alkinylgruppe, eine cyclische Alkyl- oder Alkenylgruppe, eine Aryl- oder Heteroarylgruppe ist, optional ein C-Atom dieser Gruppe X eine Gruppe R⁵ gemäß obiger Definition tragen kann.

2. Verbindung gemäß Anspruch 1, **dadurch gekennzeichnet, dass** Y ausgewählt ist aus keinem Atom, -CH₂-, -NH-(C=O)-, -(C=O)-NH-, -NR¹-, wobei R¹ wie in Anspruch 1 definiert ist.

3. Verbindung gemäß einem der Ansprüche 1 und 2, **dadurch gekennzeichnet, dass** n eine ganze Zahl zwischen 0 und 3 ist.

4. Verbindung gemäß einem der Ansprüche 1 bis 3, **dadurch gekennzeichnet, dass** m eine ganze Zahl zwischen 0 und 3 ist.

5. Verbindung gemäß einem der Ansprüche 1 bis 4, **dadurch gekennzeichnet, dass** Z eine Gruppe 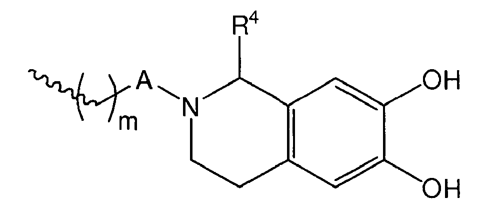 darstellt, worin m, A und R⁴ wie in Anspruch 1 definiert sind.

6. Verbindung gemäß einem der Ansprüche 1 bis 3, **dadurch gekennzeichnet, dass** dieGruppierung YZ ausgewählt ist aus

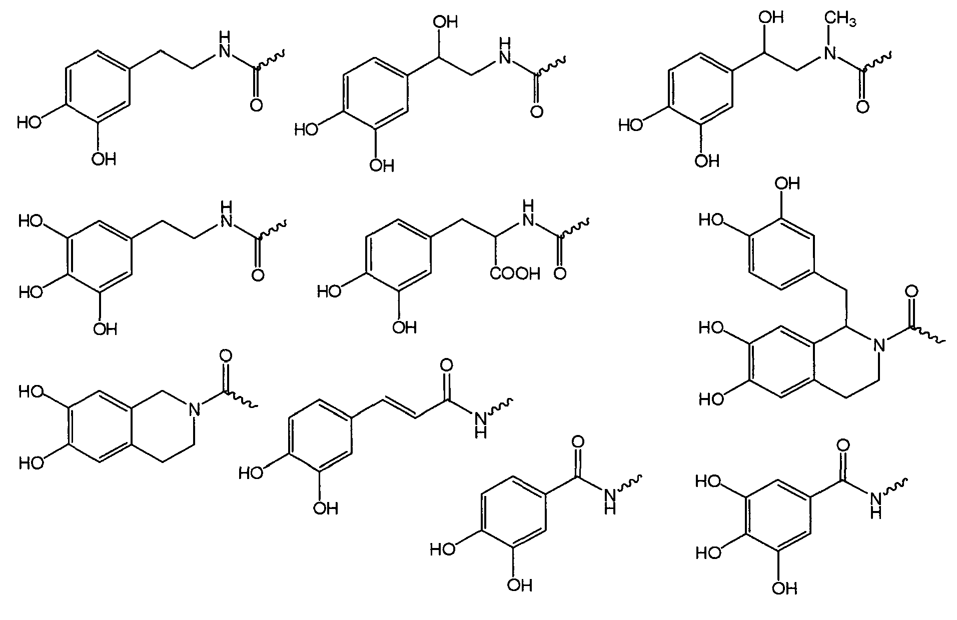

7. Verbindung gemäß einem der Ansprüche 1 bis 6, **dadurch gekennzeichnet, dass** X eine Gruppe (-CH₂)ₚ-R⁵ darstellt, wobei p eine ganze Zahl zwischen 0 und 3 darstellt und R⁵ wie in Anspruch 1 definiert ist

8. Verbindung gemäß einem der Ansprüche 1 bis 7, **dadurch gekennzeichnet, dass** X eine Gruppe -(CH₂)ₚ-R⁵ darstellt, worin R⁵ ausgewählt ist aus -H, -OH, -NH₂, -NO₂, -NH-NH₂, -NHR⁶, -NR⁶R⁷, -O-NH₂, -NH-(C=O)-C=CH, -C=CH, -N=C=S, -N=C=O, -COOH, -(C=O)H, -(C=O)R⁶ und wobei p eine ganze Zahl zwischen 0 und 3 darstellt und R⁶ und R⁷ wie in Anspruch 1 definiert sind.

9. Verfahren zur Herstellung von Verbindungen gemäß einem der Ansprüche 1 bis 8, **dadurch gekennzeichnet, dass** eine Verbindung X-C[(CH₂)ₙ-Y']₃ mit einem Reagenz Y"Z zur korrespondierenden Verbindung X-C[(CH₂)ₙ-YZ]₃ umgesetzt und das Reaktionsprodukt anschließend gereinigt wird, wobei Ad für das Adamantylgerüst und Y' für einen Precursor (Vorläufer) der Gruppe Y gemäß Formel (I) stehen und worin X, Z und n wie in Anspruch 1 definiert sind.

10. Verfahren gemäß Anspruch 9, dadurch ciekennzeichnet, dass X ein Wasserstoffatom ist.

11. Verfahren gemäß Anspruch 9, **dadurch gekennzeichnet, dass** X eine Gruppe -(CH₂)ₚ-R⁵ ist, worin p eine ganze Zahl zwischen 0 und 3 darstellt und R⁵ ausgewählt ist aus -OH, -NH₂, -NO₂, -NH-NH₂, -NHR⁶, -NR⁶R⁷, -O-NH₂, -NH-(C=O)-C≡CH, -C≡CH, -N=C=S, -N=C=O, -COOH, -(C=O)H, -(C=O)R⁶, wobei R⁶ und R⁷ wie in Formel (I) definiert sind.

12. Verfahren gemäß Anspruch 11, **dadurch gekennzeichnet, dass** die Gruppe R⁵ vor der Umsetzung mit dem Reagenz Y"Z durch eine Schutzgruppe Pg geschützt wird, so dass die Verbindung Pg-X-C[(CH₂)ₙ-Y']₃ mit einem Reagenz Y"Z zur korrespondierenden Verbindung Pg-X-C[(CH₂)ₙ-YZ]₃ umgesetzt wird.

13. Verwendung von Verbindungen gemäß einem der Ansprüche 1 bis 8 in einem Verfahren zur Funktionalisierung von Oberflächen, wobei die Funktionalisierung mittels dip and rinse erfolgt.

14. Verwendung von Verbindungen gemäß Anspruch 13, **dadurch gekennzeichnet, dass** die funktionelle Gruppe X der Verbindungen gemäß Formel (I) zuerst mit einem Effektor gekoppelt wird und danach die Funktionalisierung der Oberfläche mittels dip and rinse erfolgt.

15. Verwendung von Verbindungen gemäß Anspruch 13, **dadurch gekennzeichnet. dass** zuerst die Funktionalisierung der Oberflächen mittels dip and rinse erfolgt und danach die funktionelle Gruppe X der Verbindungen gemäß Formel (I) mit einem Effektor gekoppelt wird.

## Claims

1. Compounds according to formula (I): 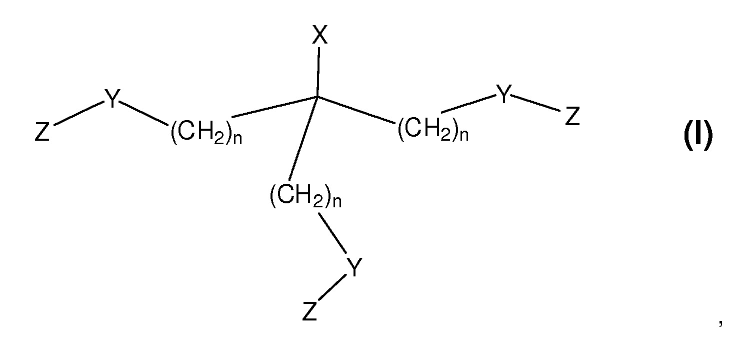 wherein
- n stands for an integer between 0 and 10,
- Y is selected from -CH₂-, -CH=CH-, -C≡C-, -O-, -S-, -S-S-, -NH-, -O- -NH-, -NH-O-, -HC=N-O-, -O-N=CH-, -NR¹-, -aryl-, -heteroaryl-, -(C=O)-, -O-(C=O)-, -(C=O)-O-, -NH-(C=O)-, -(C=O)-NH-, -NR¹-(C=O)-, -(C=O)-NR¹-, -NH-(C=O)-NH-, -NH-(C=S)-NH-, wherein R¹ stands for a linear alkyl group with 1 to 10 C atoms or for a branched or cyclic alkyl group with 3 to 10 C atoms,
- Z is selected from 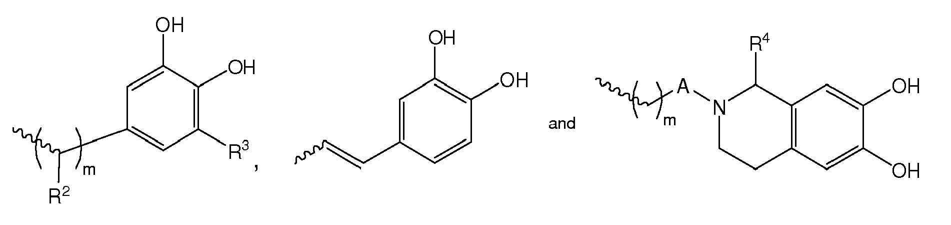 with
m = an integer between 0 and 10,
R² = -H, -OH or -COOH,
R³ = -H, -OH
A = no atom or -(C=O)-
R⁴ = -H or 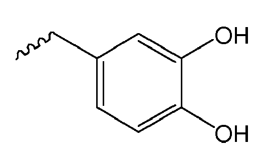 and
- X stands for a group -(CH₂)ₚ-R⁵, wherein p = 0 to 10 and R⁵ is selected from -H, -NH₂, -NO₂, -OH, -SH, -O-NH₂, -NH-NH₂, -N=C=S, -NC=O, -CH=CH₂, -C≡CH, -COOH, -(C=O)H, -(C=O)R⁶. wherein the hydroxy, thio, amino or C=O groups are optionally suitable to be protected by a protective group, -N₃, -OR⁶, -COOR⁶, -NHR⁶, -NR⁶R⁷, -CO-NHR⁶, -CONR⁶R⁷, -NH-CO-R⁶, 4-(2,5-dioxopyrrol-1-yl), wherein R⁶ and R⁷ stand independently of one another for a linear alkyl group with 1 to 10 C atoms, a linear alkenyl or alkynyl group with 2 to 10 C atoms, a branched alkyl, alkenyl or alkynyl group with 3 to 10 C atoms or
wherein, in the event that X is a branched alkyl, alkenyl and alkynyl group, a cyclic alkyl or alkenyl group, an aryl or heteroaryl group, one C atom of this group X is optionally suitable to carry one group R⁵ according to the definition above.

2. Compound according to claim 1, wherein Y is selected from no atom, -CH₂-, -NH-(C=O)-, -(C=O)-NH-, -NR¹-, wherein R¹ is defined as in claim 1.

3. Compound according to one of the claims 1 and 2, wherein n is an integer between 0 and 3.

4. Compound according to one of the claims 1 to 3, wherein m is an integer between 0 and 3.

5. Compound according to one of the claims 1 to 4, wherein Z represents a group 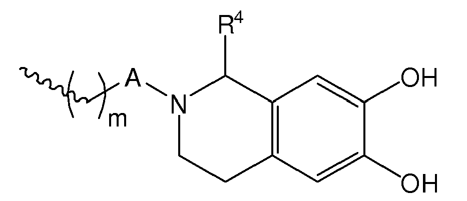 wherein m, A and R⁴ are defined as in claim 1.

6. Compound according to one of the claims 1 to 3, wherein the group YZ is selected from

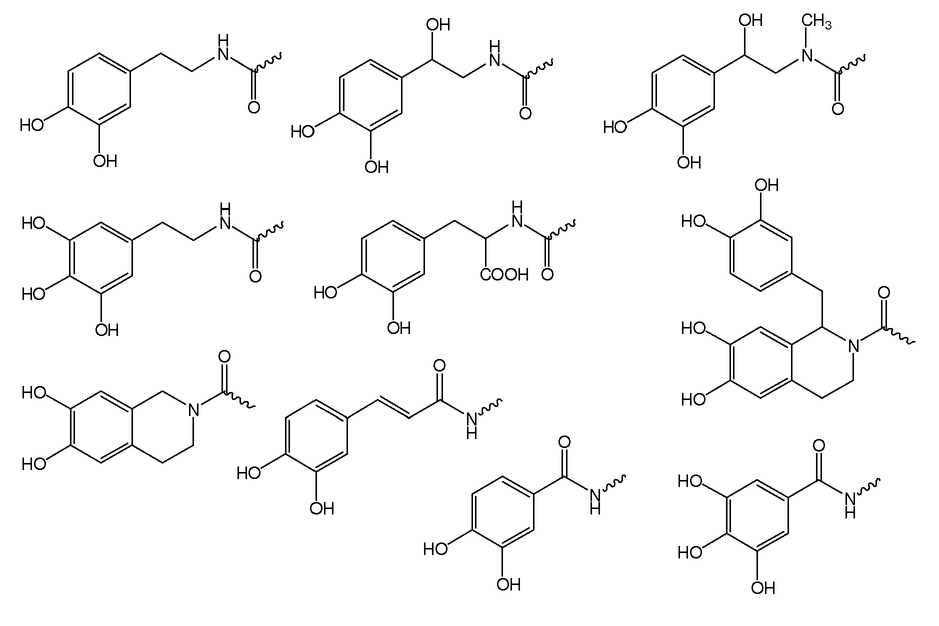

7. Compound according to one of the claims 1 to 6, wherein X represents a group (CH₂)ₚ-R⁵, wherein p represents an integer between 0 and 3, and R⁵ is defined as in claim 1.

8. Compound according to one of the claims 1 to 7, wherein X represents a group -(CH₂)ₚ-R⁵, wherein R⁵ is selected from -H, -OH, -NH₂, -NO₂, -NH-NH₂, -NHR⁶, -NR⁶R⁷, -O-NH₂, -NH-(C=O)-C≡CH, -C≡CH, -N=C=S, -N=C=O, -COOH, -(C=O)H, -(C=O)R⁶, and wherein p represents an integer between 0 and 3, and R⁶ and R⁷ are defined as in claim 1.

9. Method for the production of compounds according to one of the claims 1 to 8, wherein a compound X-C[(CH₂)ₙ-Y']₃ is reacted with a reagent Y"Z to the corresponding compound X-C[(CH₂)ₙ-YZ]₃, and the reaction product is subsequently purified, wherein Ad stands for the adamantyl skeleton and Y' for a precursor of the group Y according to formula (I), and wherein X, Z and n are defined as in formula (I).

10. Method according to claim 9, wherein X is a hydrogen atom.

11. Compound according to claim 9, wherein X is a group -(CH₂)ₚ-R⁵, wherein p represents an integer between 0 and 3, and R⁵ is selected from -OH, -NH₂, -NO₂, -NH-NH₂, -NHR⁶, -NR⁶R⁷ -O-NH₂, -NH-(C=O)-C≡CH, -C≡CH, -N=C=S, -N=C=O, -COOH, -(C=O)H, -(C=O)R⁶, wherein R⁶ and R⁷ are defined as in formula (I).

12. Method according to claim 11, wherein the group R⁵ is protected by a protective group Pg prior to the reaction with the reagent Y"Z, so that the compound Pg-X-C[(CH₂)ₙ-Y']₃ is reacted with a reagent Y"Z to the corresponding compound Pg-X-C[(CH₂)ₙ-YZ]₃.

13. Use of compounds according to one of the claims 1 to 8 within a method for the functionalisation of surfaces, wherein the functionalisation occurs by means of dip and rinse.

14. Use of compounds according to claim 13, wherein the functional group X of the compounds according to formula (I) is first coupled with an effector, and the functionalisation of the surface subsequently occurs by means of dip and rinse.

15. Use of compounds according to claim 13, wherein the functionalisation of the surfaces occurs first by means of dip and rinse, and the functional group X of the compounds according to formula (I) is subsequently coupled with an effector.

## Revendications

1. Composés selon la formule (I) : 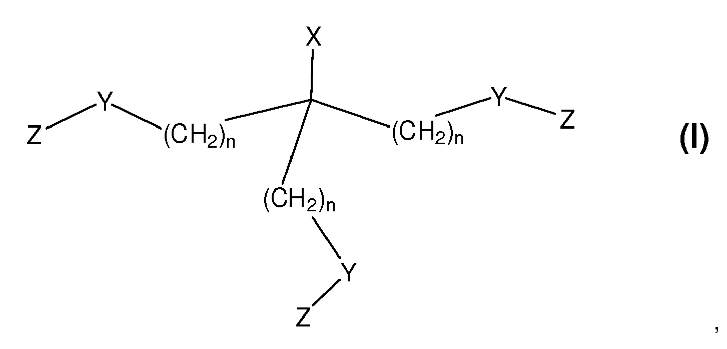 où
- n représente un nombre entier entre 0 et 10,
- Y est choisi parmi -CH₂-, -CH=CH-, -C≡C-, -O-, -S-, -S-S-, -NH-, -O- -NH-, -NH-O-, -HC=N-O-, -O-N=CH-, -NR¹-, -aryl-, -hétéroaryl-, -(C=O)-, -O-(C=O)-, -(C=O)-O-, -NH-(C=O)-, -(C=O)-NH-, -NR¹-(C=O)-, -(C=O)-NR¹-, -NH-(C=O)-NH-, -NH-(C=S)-NH-, où R¹ représente un groupe alkyle linéaire avec 1 à 10 atomes de carbone ou un groupe alkyle ramifié ou cyclique avec 3 à 10 atomes de carbone,
- Z es choisi parmi 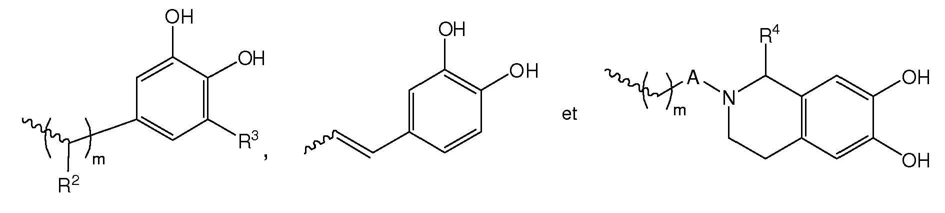 avec
m = un nombre entier entre 0 et 10,
R² = -H, -OH ou -COOH,
R³ = -H, -OH
A = pas d'atome ou -(C=O)-
R⁴ = -H ou 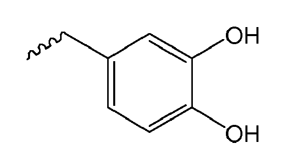 et
- X représente un groupe -(CH₂)ₚ-R⁵, où p = 0-10 et R⁵ est choisi parmi -H, -NH₂, -NO₂, -OH, -SH, -O-NH₂, -NH-NH₂, -N=C=S, -NC=O, -CH=CH₂, -C≡CH, -COOH, -(C=O)H, -(C=O)R⁶. où les groupes hydroxy, thio, amino ou C=O peuvent être optionnellement protégés par un groupe protecteur, -N₃, -OR⁶, -COOR⁶, -NHR⁶, -NR⁶R⁷, -CO-NHR⁶, -CONR⁶R⁷, -NH-CO-R⁶, 4-(2,5-dioxopyrrole-1-yl), où R⁶ et R⁷ représentent indépendamment l'un de l'autre un groupe alkyle linéaire avec 1 à 10 atomes de carbone, un groupe alcényle ou un groupe alcynyle linéaires avec 2 à 10 atomes de carbone, un groupe alkyle, alcényle ou alcynyle ramifiés avec 3 à 10 atomes de carbone ou un groupe alkyle ou alcényle cycliques avec 3 à 10 atomes de carbone, ou, dans le cas où X est un groupe alkyle, alcényle et alcynyle ramifiés, un groupe alkyle ou alcényle cycliques, un groupe aryle ou hétéroaryle, un atome de carbone de ce groupe X peut optionnellement porter un groupe R⁵ tel que défini ci-dessus.

2. Composé selon la revendication 1, **caractérisé en ce que** Y est choisi parmi aucun atome, -CH₂-, -NH-(C=O)-, -(C=O)-NH-, -NR¹-, où R¹ est tel que défini dans la revendication 1.

3. Composé selon l'une des revendications 1 et 2, **caractérisé en ce que** n est un nombre entier entre 0 et 3.

4. Composé selon l'une des revendications 1 à 3, **caractérisé en ce que** m est un nombre entier entre 0 et 3.

5. Composé selon l'une des revendications 1 à 4, **caractérisé en ce que** Z représente un groupe 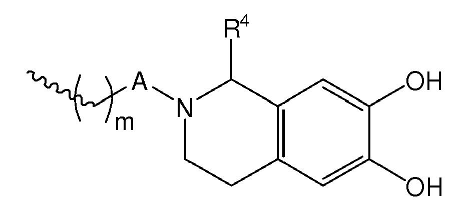 où m, A et R⁴ sont tels que définis dans la revendication 1.

6. Composé selon l'une des revendications 1 à 3, **caractérisé en ce que** le groupement YZ est choisi parmi

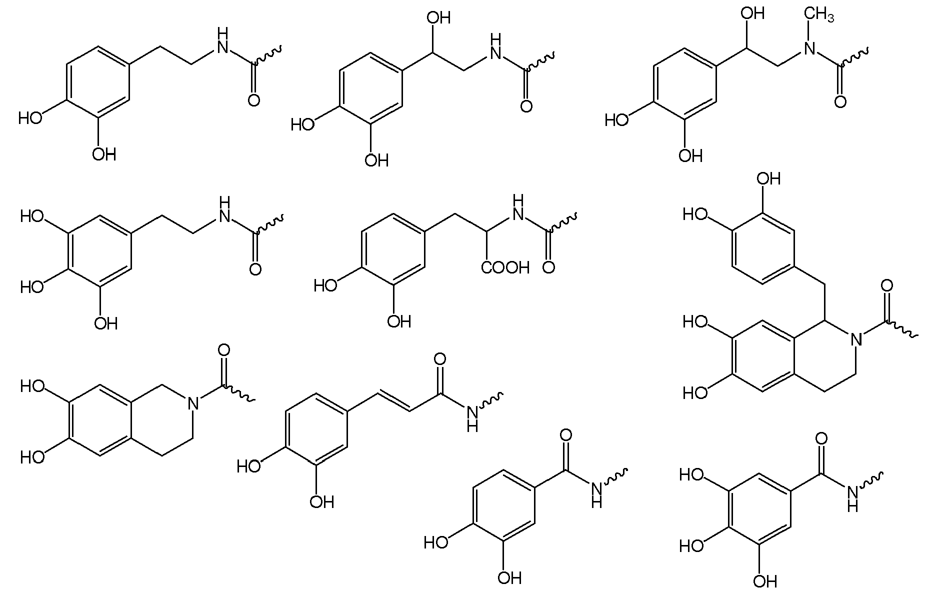

7. Composé selon l'une des revendications 1 à 6, **caractérisé en ce que** X représente un groupe (-CH₂)ₚ-R⁵, où p représente un nombre entier entre 0 et 3 et R⁵ est tel que défini dans la revendication 1.

8. Composé selon l'une des revendications 1 à 7, **caractérisé en ce que** X représente un groupe -(CH₂)ₚ-R⁵, où R⁵ est choisi parmi -H, -OH, -NH₂, -NO₂, -NH-NH₂, -NHR⁶, -NR⁶R⁷, -O-NH₂, -NH-(C=O)-C≡CH, -C≡CH, -N=C=S, -N=C=O, -COOH, -(C=O)H, -(C=O)R⁶ et où p représente un nombre entier entre 0 et 3 et R⁶ et R⁷ sont tels que définis dans la revendication 1.

9. Procédé pour la préparation de composés selon l'une des revendications 1 à 8, **caractérisé en ce que** l'on fait réagir un composé X-C[(CH₂)ₙ-Y']₃ avec un réactif Y"Z au composé correspondant X-C[(CH₂)ₙ-YZ]₃ et le produit de réaction est ensuite purifié, où Ad représente le squelette adamantyle et Y' un précurseur du groupe Y selon la formule (I) et dans laquelle X, Z et n sont tels que définis dans la revendication 1.

10. Composé selon la revendication 9, **caractérisé en ce que** X est un atome d'hydrogène.

11. Composé selon la revendication 9, **caractérisé en ce que** X est un groupe -(CH₂)ₚ-R⁵, où p représente un nombre entier entre 0 et 3 et R⁵ est choisi parmi -OH, -NH₂, -NO₂, -NH-NH₂, -NHR⁶, -NR⁶R⁷, -O-NH₂, -NH-(C=O)-C≡CH, -C≡CH, -N=C=S, -N=C=O, -COOH, -(C=O)H, -(C=O)R⁶, où R⁶ et R⁷ sont tels que définis dans la formule (I).

12. Procédé selon la revendication 11, **caractérisé en ce que** le groupe R⁵ est protégé par un groupe protecteur Pg avant la réaction avec le réactif Y"Z, de telle sorte que le composé Pg-X-C[(CH₂)ₙ-Y']₃ réagit avec un réactif Y"Z au composé correspondant Pg-X-C[(CH₂)ₙ-YZ]₃.

13. Utilisation de composés selon l'une des revendications 1 à 8 dans un procédé pour la fonctionnalisation de surfaces, où la fonctionnalisation s'effectue moyennant immersion et rinçage.

14. Utilisation de composés selon la revendication 13, **caractérisée en ce que** le groupe fonctionnel X des composés selon la formule (I) est d'abord couplé à un effecteur et ensuite la fonctionnalisation de la surface s'effectue moyennant immersion et rinçage.

15. Utilisation de composés selon la revendication 13, **caractérisée en ce que** d'abord la fonctionnalisation des surfaces s'effectue moyennant immersion et rinçage et ensuite le groupe fonctionnel X des composés selon la formule (I) est couplé à un effecteur.
